(19) **Europäisches Patentamt / European Patent Office / Office européen des brevets**

(11) **EP 4 455 936 A1**

(12) **EUROPEAN PATENT APPLICATION**
published in accordance with Art. 153(4) EPC

(43) Date of publication:
**30.10.2024 Bulletin 2024/44**

(21) Application number: **23838484.6**

(22) Date of filing: **20.04.2023**

(51) International Patent Classification (IPC):
**G06N 3/04** (2023.01)

(52) Cooperative Patent Classification (CPC):
**G16C 20/70; G16C 20/10**

(86) International application number:
**PCT/CN2023/089401**

(87) International publication number:
**WO 2024/011983 (18.01.2024 Gazette 2024/03)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **14.07.2022 CN 202210826462**

(71) Applicant: **Tencent Technology (Shenzhen) Company Limited**
**Shenzhen, Guangdong 518057 (CN)**

(72) Inventors:
• **MENG, Ziqiao**
**Shenzhen, Guangdong 518057 (CN)**
• **ZHAO, Peilin**
**Shenzhen, Guangdong 518057 (CN)**

(74) Representative: **Reddie & Grose LLP**
**The White Chapel Building**
**10 Whitechapel High Street**
**London E1 8QS (GB)**

(54) **REACTION PRODUCT PREDICTION MODEL TRAINING METHOD AND APPARATUS, APPLICATION METHOD AND APPARATUS, AND DEVICE**

(57) Embodiments of the present application disclose a reaction product prediction model training method and apparatus, an application method and apparatus, and a device. The related embodiments can be applied to scenes such as artificial intelligence, and are used for reducing the model training cost and improving the prediction precision. The method comprises: constructing a positive sample reactant set and a negative sample reactant set by means of a first auxiliary network, and calculating a reaction predicted loss value on the basis of the positive sample reactant set and the negative sample reactant set; constructing a positive sample reaction group set and a negative sample reaction group set by means of a second auxiliary network, and calculating a reaction relationship predicted loss value on the basis of the positive sample reaction group set and the negative sample reaction group set; by means of a third auxiliary network, acquiring a predicted probability value of atoms in a sample reactant existing in a main product, and an atomic label; calculating an atom predicted loss value; and training a reaction product prediction model on the basis of the reaction predicted loss value, the reaction relationship predicted loss value and the atom predicted loss value to obtain a target reaction product prediction model.

FIG. 2

## Description

RELATED APPLICATION

This application claims priority to Chinese Patent Application No. 2022108264623, filed with the China National Intellectual Property Administration on July 14, 2022 and entitled "TRAINING METHOD AND APPLICATION METHOD FOR REACTION PRODUCT PREDICTION MODEL, APPARATUS, AND DEVICE".

FIELD OF THE TECHNOLOGY

The present disclosure relates to the field of artificial intelligence technologies, and in particular, to a reaction product prediction technology.

BACKGROUND OF THE DISCLOSURE

The task of predicting organic chemical reaction products is extremely significant to fields such as computational chemistry and pharmaceuticals.

A conventional organic chemical reaction prediction method relies on common reaction templates to predict possible product structures. However, there are many types of organic chemical reactions, and new reactions are emerging as chemical researches become increasingly sophisticated. Consequently, it is difficult for the reaction templates to cover all reaction types, and the reaction templates cannot be applied to new reaction types. With the development of the deep learning technology, it is particularly important to use the deep learning technology to learn of potential reaction rules from organic chemical reaction data.

However, the currently used technology of performing deep learning based on organic chemical reaction data generally relies on a large quantity of manual labels to train reaction product prediction models. Moreover, manual label data is usually very expensive, and as a data amount of chemical reaction data increases, support from more manual labels is usually needed, which consumes a lot of labor costs and time costs, resulting in high costs for the task of predicting organic chemical reaction products.

SUMMARY

Embodiments of the present disclosure provide a training method and an application method for a reaction product prediction model, an apparatus, and a device, to implement automatic annotation on data of a sample reaction data set without relying on manual annotation, thereby reducing costs of reaction product prediction tasks, and improving accuracy of predicting a reaction product by a reaction product prediction model.

An aspect of embodiments of the present disclosure provides a method for training a reaction product prediction model, executable by a computer device, the method including:

performing vector conversion on a reaction of a sample reaction data set using an encoding network of a reaction product prediction model, to obtain a sample reactant vector and a sample reaction product vector, the sample reaction data set indicating a plurality of reactions each including a sample reactant and a sample reaction product;

constructing a positive sample reactant set and a negative sample reactant set using a first auxiliary network according to the sample reactant vector;

determining a reaction prediction lerror based on the positive sample reactant set and the negative sample reactant set, the reaction prediction error indicating accuracy for predicting whether reactants inputted to the reaction product prediction model react;

constructing a positive sample reaction pair set and a negative sample reaction pair set using a second auxiliary network according to the sample reactant vector and the sample reaction product vector;

determining a reaction relationship prediction error based on the positive sample reaction pair set and the negative sample reaction pair set, the reaction relationship prediction error indicating accuracy for predicting whether a correspondence between a reactant and a reaction product is correct by the reaction product prediction model;

predicting, using a third auxiliary network and according to the sample reactant vector and the sample reaction product vector, an atom label and a probability value of an atom in the sample reactant being present in a main product;

determining an atom prediction error based on the predicted probability value and the predicted atom label; and

training the reaction product prediction model by using the reaction prediction error, the reaction relationship prediction error, and the atom prediction error, to obtain an updated reaction product prediction model.

Another aspect of the present disclosure provides a method for applying a reaction product prediction model, executable by a computer device, the method including:

inputting an initial reactant to the updated reaction product prediction model, to output a predicted change probability of an adjacency matrix;

predicting a change amount of the adjacency matrix based on the predicted change probability of the adjacency matrix; and

determining a reaction product based on the predicted change amount of the adjacency matrix and an adjacency matrix of the initial reactant.

Another aspect of the present disclosure provides a training apparatus for a reaction product prediction model, including:

an obtaining unit, configured to perform vector conversion on a reaction of a sample reaction data set using an encoding network of a reaction product prediction model, to obtain a sample reactant vector and a sample reaction product vector, the sample reaction data set indicating a plurality of reactions each including a sample reactant and a sample reaction product,

the obtaining unit being further configured to construct a positive sample reactant set and a negative sample reactant set using a first auxiliary network according to the sample reactant vector;

a processing unit, configured to determine a reaction prediction error based on the positive sample reactant set and the negative sample reactant set the reaction prediction error indicating accuracy for predicting whether reactants inputted to the reaction product prediction model react,

the obtaining unit being configured to construct a positive sample reaction pair set and a negative sample reaction pair set using a second auxiliary network according to the sample reactant vector and the sample reaction product vector;

the processing unit being further configured to determine a reaction relationship prediction error based on the positive sample reaction pair set and the negative sample reaction pair set, the reaction relationship prediction error indicating accuracy for predicting whether a correspondence between a reactant and a reaction product is correct by the reaction product prediction model;

the obtaining unit being configured to predict, using a third auxiliary network and according to the sample reactant vector and the sample reaction product vector, an atom label and a probability value of an atom in the sample reactant being present in a main product; and

the processing unit being further configured to determine an atom prediction error based on the predicted probability value and the predicted atom label; and

a determining unit, configured to train the reaction product prediction model b using the reaction prediction error, the reaction relationship prediction error, and the atom prediction error, to obtain an updated reaction product prediction model.

Another aspect of the present disclosure provides an apparatus for applying a reaction product prediction model, including:

an obtaining unit, configured to input an initial reactant to the updated reaction product prediction model, to output a predicted change probability of an adjacency matrix;

a processing unit, configured to predict a change amount of the adjacency matrix based on the predicted change probability of the adjacency matrix; and

a determining unit, configured to determine a target reaction product based on the predicted change amount of the adjacency matrix and an adjacency matrix of the initial reactant.

Another aspect of the present disclosure provides a computer device, including: a memory, a processor, and a bus system.

The memory is configured to store a program.

The processor is configured to implement the method described in the foregoing aspects when executing the program in the memory.

The bus system is configured to connect the memory and the processor, to cause the memory and the processor to perform communication.

Another aspect of the present disclosure provides a computer-readable storage medium, including instructions, causing, when run on a computer, the computer to perform the method according to the foregoing aspects.

Another aspect of the present disclosure provides a computer program product, the computer program, when executed by a processor, implementing the method according to the foregoing aspects.

It can be learned from the foregoing technical solutions that, the embodiments of the present disclosure have the following beneficial effects:

After the sample reactant vector and the sample reaction product vector are obtained, the positive sample reactant set and the negative sample reactant set are constructed through the first auxiliary network. The reaction prediction error is determined based on the positive sample reactant set and the negative sample reactant set, and the positive sample reaction pair set and the negative sample reaction pair set are constructed through the second auxiliary network. The reaction relationship prediction error is determined based on the positive sample reaction pair set and the negative sample reaction pair set. The atom label and the predicted probability value of the atom in the sample reactant being present in the main product are obtained through the third auxiliary network, and the atom prediction error is determined based on the predicted probability value and the atom label. Then, the reaction product prediction model may be trained based on the reaction prediction error, the reaction relationship prediction error, and the atom prediction error, to obtain the updated reaction product prediction model. In the foregoing manner, based on the first auxiliary network, the second auxiliary network, and the third auxiliary network that have self-supervision properties, the positive and negative sample sets and the atom label are mined and constructed according to data characteristics of the sample reaction data set, to implement automatic annotation on data of the sample reaction data set without relying on manual annotation, to reduce costs of a task of training the reaction product prediction model. Moreover, the first auxiliary network can assist the reaction product prediction model to better learn a distance relationship between reactants, so that the reaction product prediction model can predict whether reactants can react. The second auxiliary network can help the reaction product prediction model better learn of a distance relationship between a reactant and a product, so that reaction product prediction model has a capability of predicting a correspondence between a reactant and a product. The third auxiliary network can help the reaction product prediction model better learn of a change of a key relationship between atoms in a reactant during reaction, so that the reaction product prediction model has a capability of predicting whether atoms are still present in a main product after the reaction. Assisted learning based on the auxiliary networks can improves accuracy of predicting a reaction product by the reaction product prediction model.

BRIEF DESCRIPTION OF THE DRAWINGS

FIG. 1 is a schematic architectural diagram of a reaction data control system according to an embodiment of this application.

FIG. 2 is a flowchart of an embodiment of a training method for a reaction product prediction model according to an embodiment of this application.

FIG. 3 is a flowchart of another embodiment of a training method for a reaction product prediction model according to an embodiment of this application.

FIG. 4 is a flowchart of another embodiment of a training method for a reaction product prediction model according to an embodiment of this application.

FIG. 5 is a flowchart of another embodiment of a training method for a reaction product prediction model according to an embodiment of this application.

FIG. 6 is a flowchart of another embodiment of a training method for a reaction product prediction model according to an embodiment of this application.

FIG. 7 is a flowchart of another embodiment of a training method for a reaction product prediction model according to an embodiment of this application.

FIG. 8 is a flowchart of another embodiment of a training method for a reaction product prediction model according to an embodiment of this application.

FIG. 9 is a flowchart of another embodiment of a training method for a reaction product prediction model according to an embodiment of this application.

FIG. 10 is a flowchart of another embodiment of a training method for a reaction product prediction model according to an embodiment of this application.

FIG. 11 is a flowchart of another embodiment of a training method for a reaction product prediction model according to an embodiment of this application.

FIG. 12 is a flowchart of another embodiment of a training method for a reaction product prediction model according to an embodiment of this application.

FIG. 13 is a flowchart of an embodiment of an application method for a reaction product prediction model according to an embodiment of this application.

FIG. 14 is a flowchart of another embodiment of an application method for a reaction product prediction model according to an embodiment of this application.

FIG. 15 is a schematic diagram of an auxiliary network in a training method for a reaction product prediction model according to an embodiment of this application.

FIG. 16 is a schematic diagram of a principle framework of a reaction product prediction model in a training method for a reaction product prediction model according to an embodiment of this application.

FIG. 17 is a schematic diagram of an embodiment of a training apparatus for a reaction product prediction model according to an embodiment of this application.

FIG. 18 is a schematic diagram of an embodiment of an application apparatus for a reaction product prediction model according to an embodiment of this application.

FIG. 19 is a schematic diagram of an embodiment of a computer device according to an embodiment of this application.

DESCRIPTION OF EMBODIMENTS

For convenience of understanding, terms or concepts included in the embodiments of the present disclosure are explained first.

1. Transformer

A transformer includes an encoder and a decoder, and can use a self-attention mechanism without using a sequential structure of a recurrent neural network (RNN) and a long short-term memory (LSTM), to enable a model to be trained in parallel and possess global information.

2. Contrastive learning

Contrastive learning is a self-supervised learning method configured to learn common features of a data set by making a model learn, without labels, which data points are similar or different.

3. Graph neural network

A graph neural network includes a graph convolutional network, a graph attention network, a graph auto-encoder, a graph generative network, a graph spatial-temporal network, and the like, and compared with a fully-connected layer (MLP) that is the most basic in a neural network and that is a feature matrix multiplied by a weight matrix, the graph neural network has an additional adjacency matrix.

4. Auxiliary task

An auxiliary task is an important method for helping learning of a main task in reinforcement learning. During the learning of the main task, due to sparsity of rewards and difficulty of the task, an auxiliary task may be used to help learn feature representations.

It may be understood that, in a specific implementation of this application, related data, such as a sample reaction data set, may be involved. In a case that the foregoing embodiment of the present disclosure is applied to a specific product or technology, a permission or an approval from a user needs to be obtained. In addition, collection, use, and processing of the related data need to comply with relevant laws, regulations, and standards of relevant countries and regions.

It is to be understood that the training method for a reaction product prediction model and the application method for a reaction product prediction model provided in the present disclosure may be applied to various scenarios, including, but not limited to, artificial intelligence, cloud technology, computational chemistry , pharmaceuticals, and the like, for optimizing a reaction product prediction task by training a powerful reaction product prediction model, for application to scenarios such as drug retrosynthesis authentication, scientific law mining, and pharmaceutical research and development.

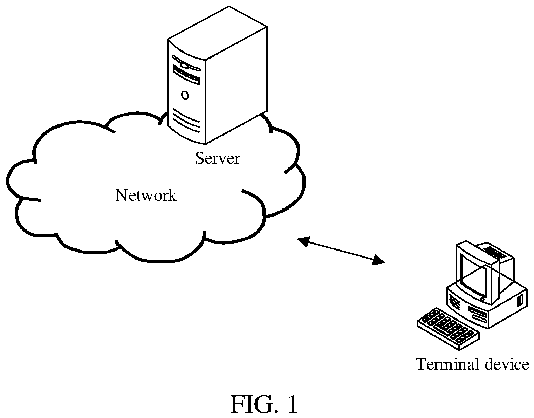

The training method for a reaction product prediction model provided in the present disclosure may be applied to a reaction data control system shown in FIG. 1. FIG. 1 is a schematic architectural diagram of a reaction data control system according to an embodiment of this application. As shown in FIG. 1, a server obtains a sample reactant vector and a sample reaction product vector based on a sample reaction data set provided by a terminal device, constructs a positive sample reactant set and a negative sample reactant set through a first auxiliary network, determines a reaction prediction error based on a positive sample reactant set and a negative sample reactant set, constructs a positive sample reaction pair set and a negative sample reaction pair set through a second auxiliary network, determines a reaction relationship prediction error based on the positive sample reaction pair set and the negative sample reaction pair set, obtains an atom label and a predicted probability value of an atom in a sample reactant being present in a main product through a third auxiliary network, and determines an atom prediction error based on the predicted probability value and the atom label, and then, may train a reaction product prediction model based on the reaction prediction error, the reaction relationship prediction error, and the atom prediction error, to obtain an updated reaction product prediction model. In the foregoing manner, based on the first auxiliary network, the second auxiliary network, and the third auxiliary network that have self-supervision properties, the positive and negative sample sets and the atom label can be mined and constructed according to data characteristics of the sample reaction data set, to implement automatic annotation on data of the sample reaction data set without relying on manual annotation, to reduce costs of a task of training the reaction product prediction model. The first auxiliary network can help the reaction product prediction model better learn of a distance relationship between reactants, so that the reaction product prediction model has a capability of predicting whether reactants can react. The second auxiliary network can help the reaction product prediction model better learn of a distance relationship between a reactant and a product, so that reaction product prediction model has a capability of predicting a correspondence between a reactant and a product. The third auxiliary network can help the reaction product prediction model better learn of a change of a key relationship between atoms in a reactant during reaction, so that reaction product prediction model has a capability of predicting whether atoms are present in a main product after the reaction. Assisted learning based on the auxiliary networks can improves accuracy of predicting a reaction product by the reaction product prediction model.

It may be understood that, FIG. 1 shows only one type of terminal device. In an actual scenario, a plurality of types of terminal devices may participate in data processing. The terminal device includes, but is not limited to, a mobile phone, a computer, an intelligent voice interaction device, an intelligent household appliance, an in-vehicle terminal, and the like. A specific quantity and a specific type depend on an actual scenario, and are not specifically limited herein. In addition, FIG. 1 shows only one server. However, there may be a plurality of servers participating in an actual scenario, particularly, in a multi-model training interaction scenario. A quantity of servers depends on an actual scenario, and is not specifically limited herein.

In the embodiments of the present disclosure including the embodiments of both the claims and specification (hereinafter referred to as "all embodiments of the present disclosure"), the server may be an independent physical server, or may be a server cluster or a distributed system formed by a plurality of physical servers, or may be a cloud server that provides basic cloud computing services such as a cloud service, a cloud database, cloud computing, a cloud function, cloud storage, a network service, cloud communication, a middleware service, a domain name service, a security service, a content delivery network (CDN), big data, and an AI platform. The terminal device may be connected to the server directly or indirectly in a wired or wireless communication manner, and the terminal device may be connected to the server to form a blockchain network, which is not limited this application.

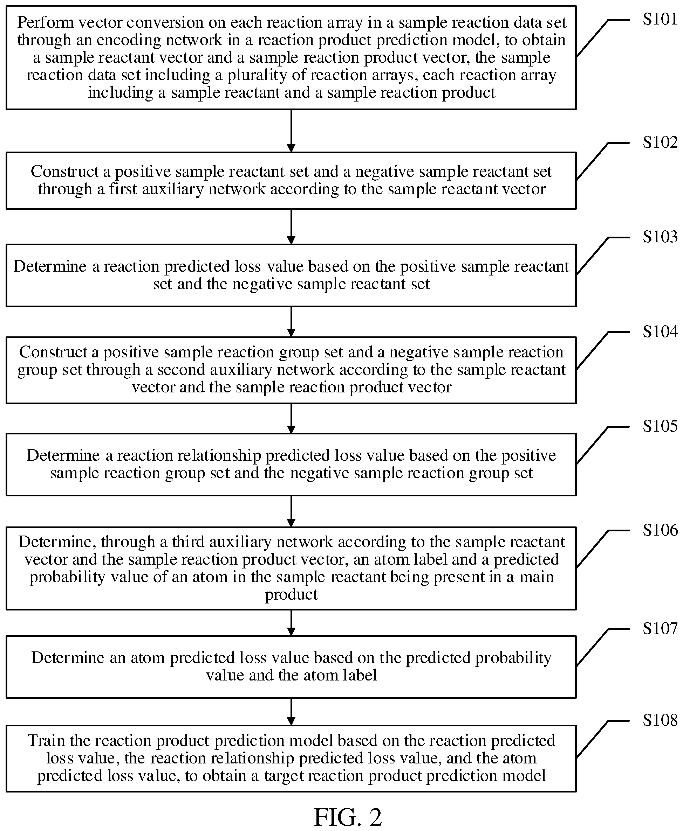

The training method for a reaction product prediction model according to the present disclosure is described below with reference to the foregoing descriptions. The method may be performed by a computer device. The computer device may be a server or a terminal device. Referring to FIG. 2, an embodiment of the training method for a reaction product prediction model in this embodiment of the present disclosure includes the following steps:

Step S101: Perform vector conversion on a reaction of a sample reaction data set using an encoding network of a reaction product prediction model, to obtain a sample reactant vector and a sample reaction product vector, the sample reaction data set indicating a plurality of reactions each including a sample reactant and a sample reaction product.

It may be understood that, the sample reaction data set includes a plurality of reaction arrays. Each reaction array may be represented as $(G^r, G^p)$. Each reaction array is configured to represent one piece of organic chemical reaction data $R_1 + R_2 + \cdots \to P_1 + P_2 + \cdots$, $G^r$ represents a series of sample reactants, and $G^p$ represents a series of products, that is, sample reaction products.

In a molecular graph G=(V, E), V represents an atom set, and a size of the set is a quantity $|V| = N$ of atoms. Each atom $v \in V$ is associated with an atom feature, including a type of the atom, chargeability, aromaticity, and the like. E represents a set of edges, and each edge is associated with a bond type, including a single bond, a double bond, a triple bond, an aromatic bond, and the like. A target of organic chemical reaction prediction is to predict a product given a reactant.

Further, due to the atom-mapping principle in organic chemical reactions, atoms in a reactant have a one-to-one correspondence with atoms in a product. Therefore, main changes before and after a reaction are changes of connections between atoms, that is, a change of an adjacency matrix A, and involved atoms do not change. Therefore, to predict a product (that is, a reaction product), only its corresponding adjacency matrix needs to be predicted, so that a structure of the entire product can be restored. The adjacency matrix used this embodiment is different from a common adjacency matrix. Further, because it is easier to predict a change, that is, $\Delta A$, of the adjacency matrix A than directly predicting A of the product, in this embodiment, an organic chemical reaction product prediction problem may be transformed into modeling a probability distribution $P(\Delta A|G^r)$.

To subsequently better model the probability distribution $P(\Delta A|G^r)$, a sample reaction data set is obtained first, and the sample reaction data set is inputted into a reaction product prediction model. Vector conversion is performed on each reaction array in the sample reaction data set through an encoding network of the reaction product prediction model, to obtain a sample reactant vector and a sample reaction product vector corresponding to the reaction array, that is, a sample reactant vector corresponding to a sample reactant in the reaction array and a sample reaction product vector corresponding to a sample reaction product in the reaction array.

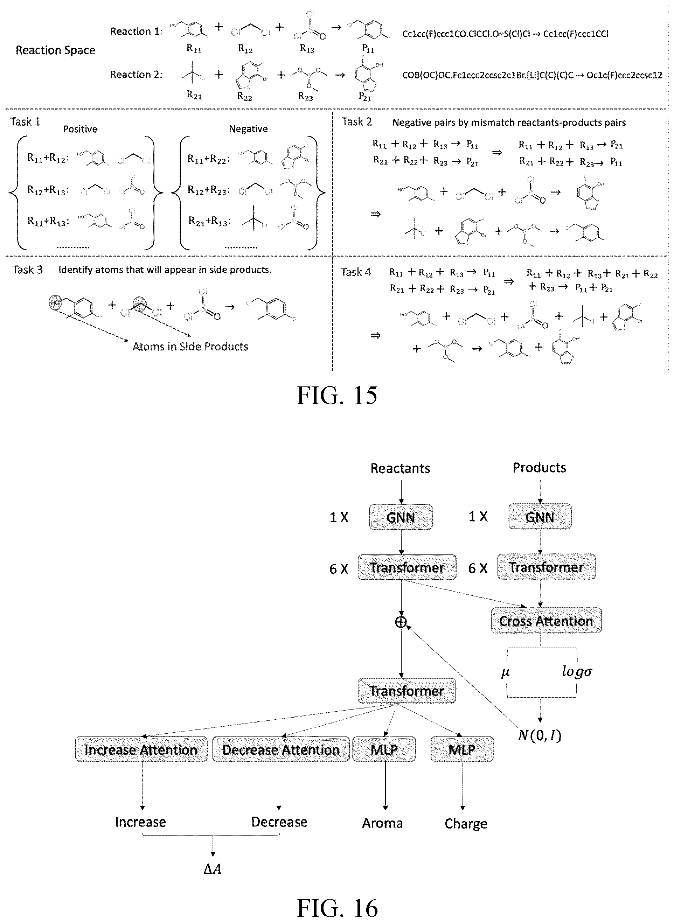

The reaction product prediction model may be specifically expressed as a VAE architecture satisfying electron transfer conservation shown in FIG. 16, and may also be expressed as another model, for example, a flow-based model, maximum likelihood training, a deep VAE, or the like, which is not specifically limited herein.

Further, if the VAE architecture satisfying electron transfer conservation shown in FIG. 16 is used as the reaction product prediction model, vector conversion may be performed on each reaction array in the sample reaction data set through a graph neural network GNN and a transformer shown in FIG. 16, to obtain a sample reactant vector and a sample reaction product vector.

Step S102: Construct a positive sample reactant set and a negative sample reactant set using a first auxiliary network according to the sample reactant vector.

In this embodiment, because in the modeling process, all predictions for a reaction product assume that a given reactant can react, but in an actual scenario, not all reactants can react, a correct reaction product prediction model is to have a capability of distinguishing whether a reactant can react. Moreover, because in embedding space, molecules that cannot react are far apart, and molecules that can react are close to each other, in this embodiment, a positive sample reactant set and a negative sample reactant set are constructed by constructing an auxiliary task for predicting whether a reactant can react, that is, a first auxiliary network, to help the reaction product prediction model learn a distance between molecules, to learn to distinguish whether a reactant has a capability of reacting, thereby enhancing the generalization capability of molecule representation. Therefore, after the sample reactant vector is obtained, the sample reactant vector is inputted into the first auxiliary network, and the positive sample reactant set and the negative sample reactant set are constructed through the first auxiliary network.

Specifically, the sample reactant vector is inputted into a task Task1, that is, the first auxiliary network, shown in FIG. 15, sample reactant vectors corresponding to any two sample reactants are sampled from a same reaction array, for example, $R_{11} + R_{12} + R_{13} \rightarrow P_{11}$, as a positive sample reactant combination, for example, $R_{11} + R_{12}$, $R_{11} + R_{13}$, and $R_{12} + R_{13}$, so that the positive sample reactant combination may be added to the positive sample reactant set positive. The positive sample reactant set includes positive sample reactant combinations, and each of the positive sample reactant combinations include two sample reactants sampled from a same reaction array.

Further, sample reactant vectors corresponding to any two sample reactants are sampled from different reaction arrays, for example, $R_{11} + R_{12} + R_{13} \rightarrow P_{11}$ and $R_{21} + R_{22} + R_{23} \rightarrow P_{21}$, as a negative sample reactant combination, for example, $R_{11} + R_{22}$, $R_{12} + R_{23}$, and $R_{21} + R_{13}$, so that the negative sample reactant combination may be added to the negative sample reactant set negative. The negative sample reactant set includes negative sample reactant combinations, and each of the negative sample reactant combinations include two sample reactants sampled from different reaction arrays.

Step S103: Determine a reaction prediction error based on the positive sample reactant set and the negative sample reactant set. The reaction prediction error indicates accuracy for predicting whether reactants inputted to the reaction product prediction model react

In all embodiments of the present disclosure, the reaction prediction error can be used to represent the reaction product prediction model's capability of distinguishing whether a reactant can react. That is, the reaction prediction error is data configured to represent the reaction product prediction model's prediction accuracy for a reaction possibility. The reaction possibility refers to whether a reactant inputted to the reaction product prediction model can react.

Specifically, after the positive sample reactant set and the negative sample reactant set are obtained, the reaction predicted error may be calculated using the following loss function formula (1):

$$L_A = \sum_{(i,j) \in D_{pos}} max(\|h_i - h_j\|_2 - \varepsilon, 0) + \sum_{(i,j) \in D_{neg}} \max\left(\gamma - \|h_i - h_j\|_2, 0\right) \quad (1)$$

where $D_{pos}$ represents the positive sample reactant set, $D_{neg}$ represents negative sample reactant set, (i, j) are configured to respectively represent a positive sample reactant combination or a negative sample reactant combination sampled from the corresponding sample reactant set, $\varepsilon$ and $\gamma$ are two margin hyperparameters, $h_i$ and $h_j$ are respectively sample reactant vector embeddings of sample reactants i and j. It may be understood that, a first term of the forward loss function formula (1) can be configured to reduce an embedding distance between positive sample molecules, and a second term thereof can be configured to increase an embedding distance between negative sample molecules.

Step S104: Construct a positive sample reaction pair set and a negative sample reaction pair set using a second auxiliary network according to the sample reactant vector and the sample reaction product vector.

In this embodiment, because a ranking of a candidate product is a piece of important information, allowing the reaction product prediction model to receive possibility scores about different candidate products, in this embodiment, an auxiliary task configured to predict whether a reactant and a product have a correct correspondence, that is, a second auxiliary network is constructed to assist the reaction product prediction model to learn a relationship between a reactant and a reaction product, and to learn a capability of distinguishing whether the reactant and the reaction product have a correct correspondence, thereby enhancing the generalization capability of molecule representation. Therefore, after the sample reactant vector and the sample reaction product vector are obtained, the sample reactant vector and the sample reaction product vector are inputted into the second auxiliary network, and the positive sample reaction pair set and the negative sample reaction pair set are constructed through the second auxiliary network.

Specifically, the sample reactant vector and the sample reaction product vector are inputted to Task2, that is, the second auxiliary network, shown in FIG. 15. Sample reactant vectors and sample reaction product vectors corresponding to all reaction arrays, where, for example, in a reaction array $R_{11} + R_{12} + R_{13} \rightarrow P_{11}$, reactants $R_{11} + R_{12} + R_{13}$ have a correct correspondence with a reaction product $P_{11}$, may be used as a positive sample reaction pair set.

Further, a negative sample reaction pair set may be constructed by mismatching a reactant and a reaction product, to make a matching score between a reactant and a reaction product having an incorrect correspondence lower. For example, in a reaction array $R_{11} + R_{12} + R_{13} \rightarrow P_{11}$, reactants $R_{11} + R_{12} + R_{13}$ and a reaction product $P_{11}$ have a correct correspondence, and in another reaction array $R_{21} + R_{22} + R_{23} \rightarrow P_{21}$, reactants $R_{21} + R_{22} + R_{23}$ and a reaction product $P_{21}$ have a correct correspondence. A negative sample reaction pair set including incorrect correspondences between reactants and reaction products, such as $R_{11} + R_{12} + R_{13} \rightarrow P_{21}$ and $R_{21} + R_{22} + R_{23} \rightarrow P_{11}$, may be obtained by mismatching the reactants and the reaction products.

Step S105: Determine a reaction relationship prediction error based on the positive sample reaction pair set and the negative sample reaction pair set. The reaction relationship prediction error indicates accuracy for predicting whether a correspondence between a reactant and a reaction product is correct by the reaction product prediction model.

In all embodiments of the present disclosure, the reaction relationship prediction error can be used to represent the reaction product prediction model's capability of distinguishing whether a reactant has a correct correspondence with a reaction product. That is, the reaction relationship prediction error is data configured to represent the reaction product prediction model's prediction accuracy for a reaction relationship. The reaction relationship refers to a correspondence between a reactant and a reaction product. The reaction relationship prediction error herein and the reaction prediction error mentioned in step 103 above are two parallel errors. Both can be configured in the subsequent steps to train the reaction product prediction model. However, there is no necessary association between the two, and the two represent different information. The reaction prediction error is configured to represent the reaction product prediction model's capability of identifying whether a reaction can occur between reactants. The reaction relationship prediction error is used to represent the reaction product prediction model's capability of identifying whether there is a correct correspondence between a reactant and a reaction product.

Specifically, after the positive sample reaction pair set and the negative sample reaction pair set are obtained, the reaction relationship prediction error may be calculated using the following loss function formula (2):

$$L_B = \frac{1}{|B|}\sum_i max(\left\| G_\theta(\{h_r\}_{r \in R_i}) - G_\varphi\left(\{h_p\}_{p \in P_i}\right) \right\|_2 - \varepsilon, 0) + \frac{1}{|B|(|B|-1)}\sum_{i \neq j} max\left(\gamma - \right.$$

$$\left. \left\| G_\theta(\{h_r\}_{r \in R_i}) - G_\varphi\left(\{h_p\}_{p \in P_j}\right) \right\|_2, 0\right) \quad (2)$$

where $G_\theta$ is a reactant network, $G_\varphi$ is a reaction product network, $G_\theta$ and $G_\varphi$ have different parameters of the same architecture, and both the networks satisfy permutation invariance. $h_r$ is a feature vector embedding corresponding to a reactant atom, $h_p$ is a feature vector embedding corresponding to a reaction product atom, are $\varepsilon$ and $\gamma$ are two margin hyperparameters. A first term of the loss function is configured to reduce a distance between a sample reactant vector and a sample reaction product vector of the same reaction array, that is, the positive sample reaction pair set, and a second term thereof is configured to push a sample reactant vector and a sample reaction product vector of a mismatched reaction array, that is, the negative sample reaction pair set, away from each other. It may be understood that, because a task may be constructed online during model training, a size of a sample of the objective function may be set to B.

Step S106: Predict, using a third auxiliary network and according to the sample reactant vector and the sample reaction product vector, an atom label and a probability value of an atom in the sample reactant being present in a main product.

In this embodiment, because side products are often ignored in reaction data in massive data sets, for example, the disclosed data set USPTO-480K, it is easy to increase a prediction error of a reaction product. Therefore, in this embodiment, a third auxiliary network configured to predict whether an atom is present in a main product may be added to the modeling process, to supplement information about some side products to some extent, thereby improving the capability of predicting the reaction product. Therefore, after the sample reactant vector and the sample reaction product vector are obtained, the sample reactant vector and the sample reaction product vector are inputted into the third auxiliary network, and an atom label and a predicted probability value of an atom in the sample reactant being present in a main product are determined through the third auxiliary network.

Specifically, the sample reactant vector and the sample reaction product vector are inputted to a task Task3, that is, the third auxiliary network, shown in FIG. 15. Prediction is performed on a sample reactant through the third auxiliary network, to obtain a predicted probability value of an atom in the sample reactant being present in a main product. In addition, an atom in the sample reactant is compared with an atom in the sample reaction product based on the sample reactant vector and the sample reaction product vector, to obtain an atom comparison result, and an atom label is determined based on the atom comparison result.

Step S107: Determine an atom prediction error based on the predicted probability value and the predicted atom label.

In all embodiments of the present disclosure, the atom prediction error can be used to represent a capability of the reaction product prediction model to distinguish whether an atom is still in the main product after the reaction.

Specifically, after the predicted probability value and the atom label are obtained, the atom prediction error may be calculated using the following loss function formula (3):

$$L_C = \frac{1}{|B|}\sum_i\sum_j y_j \log s_j + (1 - y_j)\log(1 - s_j) \quad (3)$$

where $y_j \in \{0,1\}$ represents an atom label of an atom $j$, indicating whether the atom is in the main product after the reaction, and $s_j \in [0,1]$ represents a predicted probability value of the based on an atom embedding.

Step S108: Train the reaction product prediction model by using the reaction prediction error, the reaction relationship prediction error, and the atom prediction error, to obtain an updated reaction product prediction model.

Specifically, when the reaction prediction error, the reaction relationship prediction error, and the atom prediction error are obtained, a multi-task learning training method may be used to directly add objective functions, such as the reaction prediction error, the reaction relationship prediction error, and the atom prediction error, to a total objective function in a weighted manner, and then, parameter adjustment is performed on the reaction product prediction model based on the total objective function value. Specifically, parameter adjustment may be performed using an iterative method of backpropagation or another method, which is not specifically limited herein, to obtain the updated reaction product prediction model.

According to another aspect, in this embodiment, the first auxiliary network, the second auxiliary network, and the third auxiliary network may be pre-trained respectively based on the reaction prediction error, the reaction relationship prediction error, and the atom prediction error by using a pre-training policy, and then, the pre-trained networks are migrated to the reaction product prediction model to obtain the updated reaction product prediction model.

In this embodiment of this application, a training method for a reaction product prediction model is provided. In the foregoing manner, based on the first auxiliary network, the second auxiliary network, and the third auxiliary network that have self-supervision properties, the positive and negative sample sets and the atom label can be mined and constructed according to data characteristics of the sample reaction data set, to implement automatic annotation on data of the sample reaction data set without relying on manual annotation, to reduce costs of a task of training the reaction product prediction model. The first auxiliary network can help the reaction product prediction model better learn of a distance relationship between reactants, so that the reaction product prediction model has a capability of predicting whether reactants can react. The second auxiliary network can help the reaction product prediction model better learn of a distance relationship between a reactant and a product, so that reaction product prediction model has a capability of predicting a correspondence between a reactant and a product. The third auxiliary network can help the reaction product prediction model better learn of a change of a key relationship between atoms in a reactant during reaction, so that reaction product prediction model has a capability of predicting whether atoms are present in a main product after the reaction. Assisted learning based on the auxiliary networks can improves accuracy of predicting a reaction product by the reaction product prediction model.

Data regarding a group of reactant molecules is inputted to the updated reaction product prediction model, and the model outputs data regarding a target product molecule. The model can predict a candidate product by determining whether bond location connection between reactants changes and determining whether electrons contained in the reactants are rearranged. Based on a new reactant indicated by the data inputted to the model and the predicted candidate product described above, a new reaction can be predicted, thereby greatly improving the efficiency of researching and development of new drugs. The data regarding the group of reactant molecules includes: bond energy, a bond length, a bond angle, a bond level, and so on.

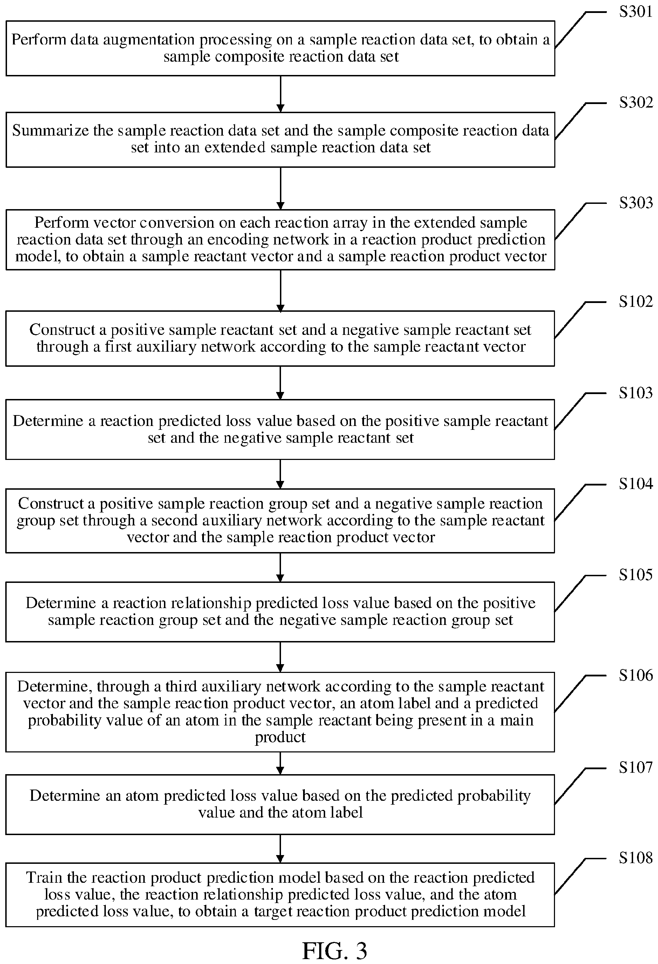

Optionally, based on the embodiment corresponding to FIG. 2, in another optional embodiment of the training method for a reaction product prediction model provided in this embodiment of this application, as shown in FIG. 3, before the performing vector conversion on each reaction array in a sample reaction data set through an encoding network in a reaction product prediction model, to obtain a sample reactant vector and a sample reaction product vector in step S101, the method further includes: step S301 to step S302; and step S101 includes step S303.

Step S301: Perform data augmentation processing on the sample reaction data set, to obtain a sample composite reaction data set.

Step S302: Combine the sample reaction data set and the sample composite reaction data set to form an extended sample reaction data set.

Step S303: Perform vector conversion on a reaction in the extended sample reaction data set using the encoding network of the reaction product prediction model, to obtain the sample reactant vector and the sample reaction product vector.

In this embodiment, although a common reaction product prediction model can predict a simple chemical reaction with, for example, one reactant or two or three reactants, it is difficult to predict a chemical reaction with more reactants or a complex chemical reaction. Based on this, in this embodiment, data augmentation is performed on the sample reaction data set during the modeling, to the sample composite reaction data set, and the sample reaction data set and the sample composite reaction data set are summarized into the extended sample reaction data set; and then, the extended sample reaction data set is inputted to the reaction product prediction model, and vector conversion is performed on each reaction array in the extended sample reaction data set through the encoding network of the reaction product prediction model, to obtain the sample reactant vector and the sample reaction product vector. In this way, not only the sample reaction data set can be expanded, but also the complexity of the sample reaction data set can be enhanced to some extent, to help the reaction product prediction model learn of the capability of predicting a complex chemical reaction, thereby improving the accuracy of predicting a reaction product to some extent.

Specifically, as indicated by a task Task4 shown in FIG. 15, data augmentation processing is performed on the sample reaction data set. Specifically, two reaction arrays may be randomly selected from the sample reaction data set, sample reactants in the two selected reaction arrays are combined, to obtain a sample composite reactant, sample reaction products in the two selected reaction arrays are combined to obtain a sample composite reaction product, and then, a composite reaction array is obtained based on the sample composite reactant and the sample composite reaction product, and a sample composite reaction data set is constructed based on the composite reaction array. Alternatively, another augmentation method may be adopted, which is not specifically limited herein. For example, reactants $R_{11} + R_{12} + R_{13}$ and a reaction product $P_{11}$ in one reaction array $R_{11} + R_{12} + R_{13} \rightarrow P_{11}$ are combined with reactants $R_{21} + R_{22} + R_{23}$ and a reaction product $P_{21}$ in another reaction array $R_{21} + R_{22} + R_{23} \rightarrow P_{21}$, to obtain a composite reaction array, for example, $R_{11} + R_{12} + R_{13} + R_{21} + R_{22} + R_{23} \rightarrow P_{11} + P_{21}$.

Further, the obtained sample reaction data set and the obtained sample composite reaction data set are summarized into an extended sample reaction data set, to facilitate subsequently utilizing the extended sample reaction data set to train the reaction product prediction model, to help the reaction product prediction model to learn of the capability of predicting a complex chemical reaction. That is, the extended sample reaction data set is inputted into the reaction product prediction model, and vector conversion is performed on each reaction array in the extended sample reaction data set through the encoding network of the reaction product prediction model to obtain the sample reactant vector and the sample reaction product vector, so that the reaction product prediction model can be trained based on the sample reactant vector and the sample reaction product vector by combining the first auxiliary network, the second auxiliary network, and the third auxiliary network.

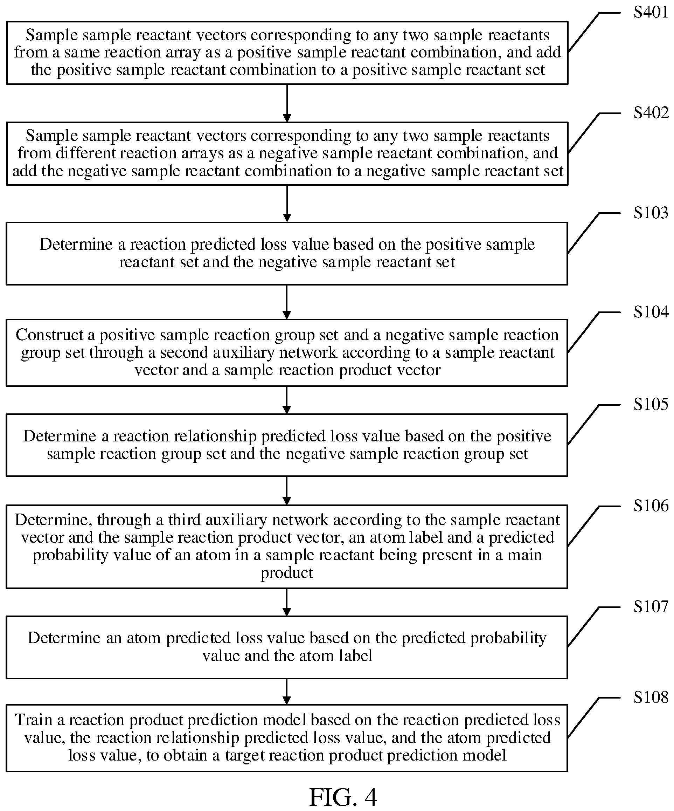

Optionally, based on the embodiment corresponding to FIG. 2 or FIG. 3, in another optional embodiment of the training method for a reaction product prediction model provided in this embodiment of this application, as shown in FIG. 4, the constructing a positive sample reactant set and a negative sample reactant set through a first auxiliary network according to the sample reactant vector in step S102 includes the following steps:

Step S401: Sample the sample reactant vectors corresponding to any two sample reactants from a same reaction array as positive sample reactants, and add the positive sample reactants to the positive sample reactant set.

Step S402: Sample the sample reactant vectors corresponding to any two sample reactants from different reaction arrays as negative sample reactants, and add the negative sample reactants to the negative sample reactant set. According to all embodiments of the present disclosure, in a scenario of researching and developing drugs, bond energy, a bond length, a bond angle and a bond level of the sample reactants are measured.

Specifically, in embedding space, molecules that cannot react are far apart, and molecules that can react are close to each other. Therefore, after a sample reactant vector corresponding to each reaction array is obtained, according to chemical reaction data, for example,

$$G_1^{R_i} + G_2^{R_i} + \cdots \rightarrow P_1^{P_i} + P_2^{P_i} + \cdots$$ , where $G_1^{R_i}$, $G_2^{R_i}$ , and the like are configured to represent sample reactants, $P_1^{P_i}$, $P_2^{P_i}$ , and the like are configured to represent sample reaction products, reactant combination data is constructed by combining reactants pairwise.

Further, as shown in FIG. 15, for convenience of expression, in this embodiment, given a batch of reaction data B, two spaces are constructed, where one is a positive sample space, that is, the positive sample reactant set, and the other is a negative sample space, that is, the negative sample reactant set. The sample reactant vector is inputted into the task Task1 shown in FIG. 15, that is, the first auxiliary network. A positive sample reactant set Dpos is constructed according to reaction data given in chemical data B. That is, sample reactant vectors corresponding to any two sample reactants in the same reaction array are combined. Assuming that each reaction has R reactants, R(R-1) positive sample reactant combinations can be obtained. A negative sample reactant set Dneg may be obtained by combining sample reactants from different reaction arrays. That is, sample reactant vectors corresponding to any two sample reactant sampled from different reaction arrays are combined.

For example, as shown in FIG. 15, sample reactant vectors corresponding to any two sample reactants are sampled from a same reaction array, for example, $R_{11} + R_{12} + R_{13} \rightarrow P_{11}$, as a positive sample reactant combination, for example, $R_{11} + R_{12}, R_{11} + R_{13}$, and $R_{12} + R_{13}$, so that a positive sample reactant set positive can be obtained through summarization.

Further, any two sample reactant vectors are sampled from different reaction arrays, for example, $R_{11} + R_{12} + R_{13} \rightarrow P_{11}$ and $R_{21} + R_{22} + R_{23} \rightarrow P_{21}$, as a negative sample reactant combination, for example, $R_{11} + R_{22}, R_{12} + R_{23}$,

and $R_{21} + R_{13}$, so that a negative sample reactant set negative can be obtained through summarization.

In this way, by constructing the positive sample reactant set and the negative sample reactant set in the foregoing way, the first auxiliary network can better help the reaction product prediction model learn of a distance relationship between reactants, so that the reaction product prediction model can predict more accurately whether a reactant can react.

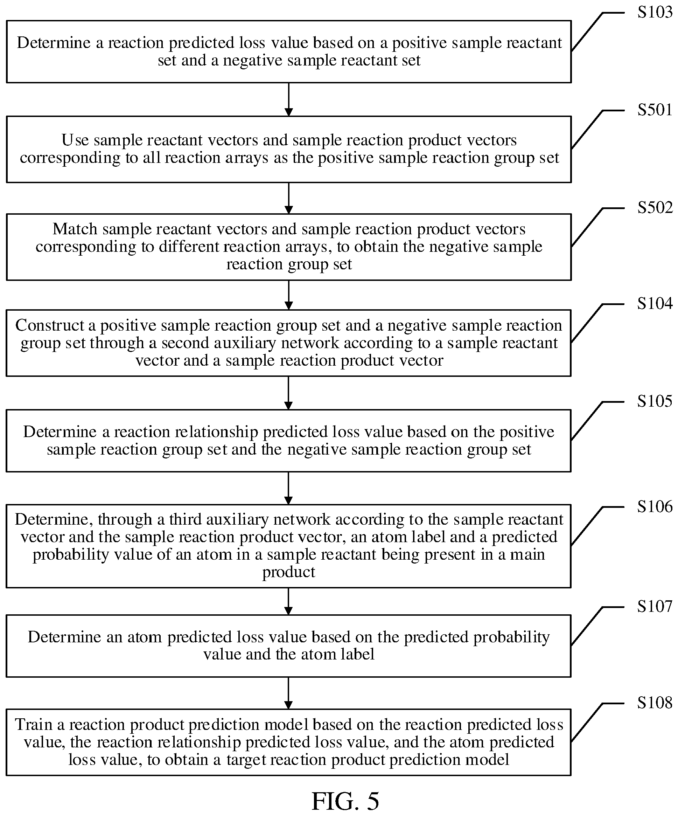

Optionally, based on the embodiment corresponding to FIG. 2 or FIG. 3, in another optional embodiment of the training method for a reaction product prediction model provided in this embodiment of this application, as shown in FIG. 5, the constructing a positive sample reaction pair set and a negative sample reaction pair set through a second auxiliary network according to the sample reactant vector and the sample reaction product vector in step S104 includes the following steps:

Step S501: Use the sample reactant vectors and the sample reaction product vectors corresponding to all the reaction arrays as the positive sample reaction pair set.
Step S502: Match the sample reactant vectors and the sample reaction product vectors corresponding to different reaction arrays, to obtain the negative sample reaction pair set.

Specifically, a batch of chemical reaction data $B = \{R_1 \rightarrow P_1, R_2 \rightarrow P_2, ... \}$ may be given, and a given reactant, for example, $R_1$ and a reaction product $P_1$ are combined as a positive sample reaction group, that is, $R_1 \rightarrow P_1$. That is, chemical reaction data B may be used as a positive sample reaction pair set. Specifically, the sample reactant vector and the sample reaction product vector are inputted to Task2, that is, the second auxiliary network, shown in FIG. 15. Sample reactant vectors and sample reaction product vectors corresponding to all reaction arrays, where, for example, in a reaction array $R_{11} + R_{12} + R_{13} \rightarrow P_{11}$, reactants $R_{11} + R_{12} + R_{13}$ have a correct correspondence with a reaction product $P_{11}$, may be used as a positive sample reaction group in the positive sample reaction pair set.

Further, the negative sample reaction pair set is constructed through combinations of reactants and reaction products in mismatched chemical reaction data $B = \{R_1 \rightarrow P_1, R_2 \rightarrow P_2, ... \}$, and the sample reactant vector and the sample reaction product vector are mismatched, to obtain a negative sample reaction group, and make a matching score between a reactant and a reaction product having an incorrect correspondence lower. For example, as shown in FIG. 15, in a reaction array $R_{11} + R_{12} + R_{13} \rightarrow P_{11}$, reactants $R_{11} + R_{12} + R_{13}$ and a reaction product $P_{11}$ have a correct correspondence, and in another reaction array $R_{21} + R_{22} + R_{23} \rightarrow P_{21}$, reactants $R_{21} + R_{22} + R_{23}$ and a reaction product $P_{21}$ have a correct correspondence. A negative sample reaction group including incorrect correspondences between reactants and reaction products, such as $R_{11} + R_{12} + R_{13} \rightarrow P_{21}$ and $R_{21} + R_{22} + R_{23} \rightarrow P_{11}$, may be obtained by mismatching the reactants and the reaction products.

In this way, by constructing the positive sample reaction pair set and the negative sample reaction pair set in the foregoing way, the second auxiliary network can better help the reaction product prediction model learn a distance relationship between a reactant and a product, so that the reaction product prediction model can predict more accurately a correspondence between a reactant and a product.

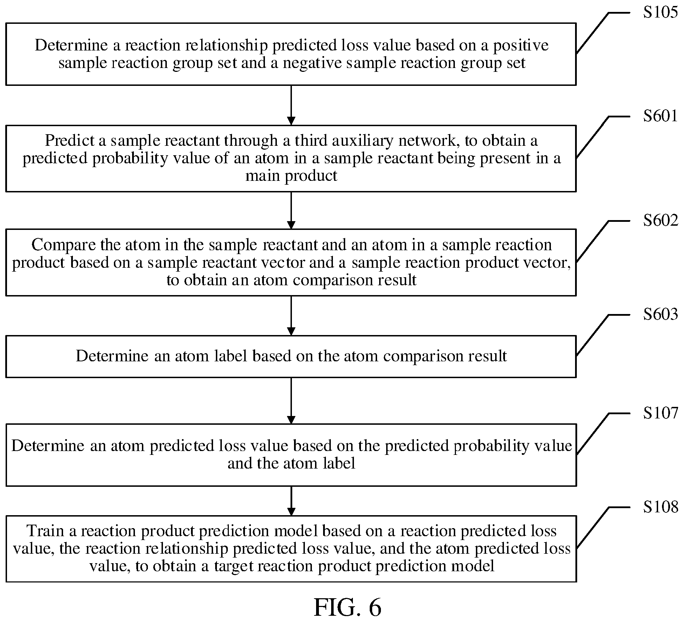

Optionally, based on the embodiment corresponding to FIG. 2 or FIG. 3, in another optional embodiment of the training method for a reaction product prediction model provided in this embodiment of this application, as shown in FIG. 6, the determining, through a third auxiliary network according to the sample reactant vector and the sample reaction product vector, an atom label and a predicted probability value of an atom in the sample reactant being present in a main product in step S106 includes the following steps:

Step S601: Predict the sample reactant using the third auxiliary network, to obtain the probability value of the atom in the sample reactant being present in the main product.

Step S602: Compare the atom in the sample reactant with an atom in the sample reaction product based on the sample reactant vector and the sample reaction product vector, to obtain an atom comparison result.

Step S603: Determine the atom label based on the atom comparison result. The reaction product includes a main product and a side product; and a content percentage of the main product is higher than a content percentage of the side product.

In this embodiment, because predicting whether an atom is present in a main product and predicting a reaction center are two tasks that are not equivalent to each other, by predicting the reaction center, only which bonds are broken can be learned of, and which one of two atoms connected by a bond is present in a side product cannot be learned of, the third auxiliary network configured to predict whether an atom is present in a main product allows the reaction product prediction model to learn of relative importance of the atom in the reaction. Therefore, prediction may be performed on a sample reactant through the third auxiliary network, to obtain a predicted probability value of an atom in the sample reactant being present in a main product. In addition, an atom in the sample reactant is compared with an atom in the sample reaction product based on the sample reactant vector and the sample reaction product vector, so that a corresponding atom comparison result can be obtained. Then, an atom label may be determined based on the atom comparison result.

Specifically, a sample reactant is predicted through the third auxiliary network, to obtain a predicted probability value of an atom in the sample reactant being present in a main product. Specifically, the task of predicting whether the atom is present in the main product can be regarded as known information that is used as a masking matrix to mask side product information, so that the reaction product prediction model can only pay attention to information about the main product, and actually, the masking matrix is also content that the reaction product prediction model is to predict.

Further, a manner of obtaining the atom label, which is a ground-truth label, may be based on sample reactant vector and sample reaction product vector. Atoms of the sample reactant are compared with atoms of the sample reaction product. An atom lost by the reactant, compared with the reaction product, is marked as 0, while an atom still remaining in the reaction product is marked as 1. Another labeling method may also be used, which is not specifically limited herein.

In this way, in the foregoing manner, the third auxiliary network can better help the reaction product prediction model learn of a change of a bond relationship between atoms of a reactant during a reaction, so that the reaction product prediction model can predict more accurately whether an atom is present in the main product after the reaction.

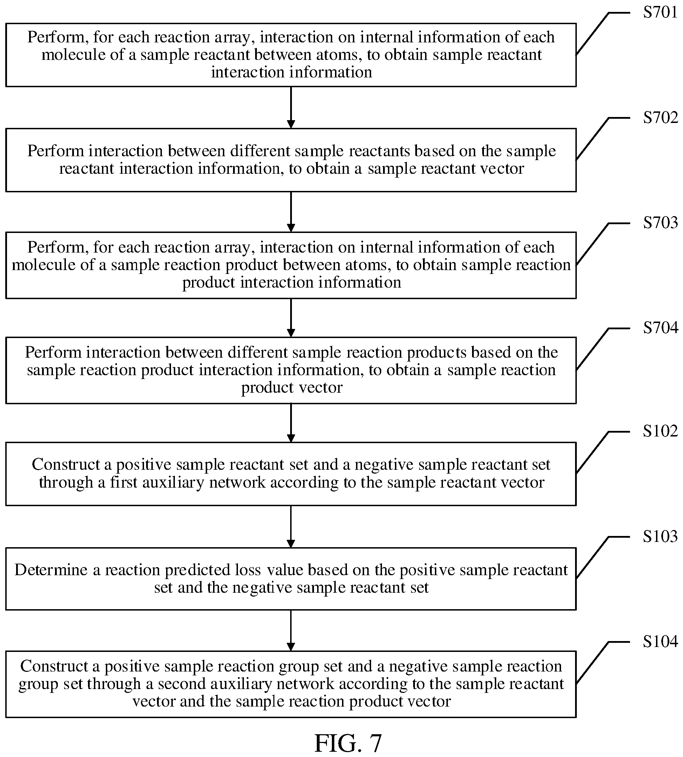

Optionally, based on the embodiment corresponding to FIG. 2 or FIG. 3, in another optional embodiment of the training method for a reaction product prediction model provided in this embodiment of this application, as shown in FIG. 7, the performing vector conversion on each reaction array in a sample reaction data set through an encoding network in a reaction product prediction model, to obtain a sample reactant vector and a sample reaction product vector in step S101 further includes the following steps:

Step S701: Perform, for each reaction, interaction on internal information of each molecule of the sample reactant between atoms, to obtain sample reactant interaction information.

Step S702: Perform interaction between different sample reactants based on the sample reactant interaction information, to obtain the sample reactant vector.

Step S703: Perform, for each reaction, interaction on internal information of each molecule of the sample reaction product between atoms, to obtain sample reaction product interaction information.

Step S704: Perform interaction between different sample reaction products based on the sample reaction product interaction information, to obtain the sample reaction product vector.

Specifically, when the VAE architecture satisfying electron transfer conservation shown in FIG. 16 is used as the reaction product prediction model, in this embodiment, the encoding network performing vector conversion on a reaction in the sample reaction data set may include a graph neural network GNN and a transformer.

The transformer includes an encoder and a decoder, and can use a self-attention mechanism without using a sequential structure of a recurrent neural network (RNN) and a long short-term memory L(STM), to enable a model to be trained in parallel and possess global information.

The graph neural network GNN includes a graph convolutional network, a graph attention network, a graph auto-encoder, a graph generative network, a graph spatial-temporal network, and the like, and compared with a fully-connected layer (MLP) that is the most basic in a neural network and that is a feature matrix multiplied by a weight matrix, the graph neural network has an additional adjacency matrix.

Therefore, interaction may be first performed on internal information of each molecule between atoms through the GNN by using the following formula (4), that is, for each reaction array, interaction is performed on internal information of each molecule of a sample reactant between atoms, to obtain sample reactant interaction information, and interaction is then performed on the information through the transformer between different reactants, that is, interaction is performed between different sample reactants based on the sample reactant interaction information, to obtain the sample reactant vector:

$$h^R = Transformer(GNN(G^R)) \quad (4)$$

where $h^R$ is configured to represent the sample reactant vector, and $G^R$ is configured to represent the sample reactant.

Likewise, interaction may be first performed on internal information of each molecule between atoms through the GNN by using the following formula (5), that is, for each reaction array, interaction is performed on internal information of each molecule of a sample reaction product between atoms, to obtain sample reaction product interaction information, and interaction is then performed on the information through the transformer between different reaction products, that is, interaction is performed between different sample reaction products based on the sample reaction product interaction information, to obtain the sample reaction product vector:

$$h^P = Transformer(GNN(G^P)) \quad (5)$$

where $h^P$ is configured to represent the sample reaction product vector, and $G^P$ is configured to represent the sample reaction product.

In this way, obtaining the sample reactant vector and the sample reaction product vector through the foregoing manner can ensure that the obtained sample reactant vector and the obtained sample reaction product vector can more accurately reflect features of the corresponding sample reactant and the corresponding sample reaction product, and further, help the first auxiliary network, the second auxiliary network, and the third auxiliary network subsequently better learn of related capabilities based on the sample reactant vector and the sample reaction product vector, thereby improving the performance of the trained reaction product prediction model.

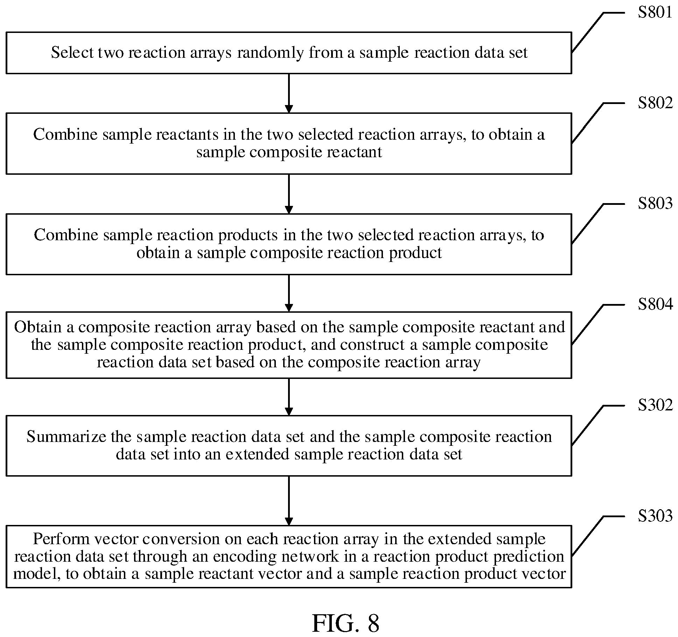

Optionally, based on the embodiment corresponding to FIG. 3, in another optional embodiment of the training method for a reaction product prediction model provided in this embodiment of this application, as shown in FIG. 8, the performing data augmentation processing on the sample reaction data set, to obtain a sample composite reaction data set, in step S301 includes the following steps:

Step S801: Select two reactions from the sample reaction data set.

Step S802: Combine sample reactants in the two selected reactions, to obtain a sample composite reactant.

Step S803: Combine sample reaction products in the two selected reactions, to obtain a sample composite reaction product.

Step S804: Generate a composite reaction based on the sample composite reactant and the sample composite reaction product, and construct the sample composite reaction data set based on the composite reaction.

In this embodiment, performing data augmentation processing on the sample reaction data set may be randomly selecting two reaction arrays from the sample reaction data set, combining sample reactants in the two selected reaction arrays to obtain a sample composite reactant, in addition, combining sample reaction products in the two selected reaction arrays to obtain a sample composite reaction product, then, obtaining a composite reaction array based on the sample composite reactant and the sample composite reaction product, and constructing a sample composite reaction data set according to the composite reaction array. Although a completely accurate chemical reaction cannot be obtained through combination according to the data augmentation method, for example, between new reactant combinations, reactions may no longer be performed between A and B and between E and F, but between A and E and between B and F, or may be performed between other combinations, or even if reactions are still performed between A and B and between E and F, only intermediate products are obtained, and can be further reacted to obtain a final product, that is, new reactions obtained through random combination using the data augmentation method may be not accurate, the generalization capability of the reaction product prediction model can still be enhanced. In addition, there may be incorrect reaction data (for example, an intermediate product instead of a final product is given as a ground-truth product) in the commonly used USPTO-480K data. Therefore, inaccurate new reaction combinations obtained using the foregoing data augmentation method have small impact on the prediction accuracy of the reaction product prediction model, so that the sample composite reaction data set obtained after the data augmentation and the original sample reaction data set may be summarized to obtain through extension an extended sample reaction data set. The extended sample reaction data set is subsequently applied to parameter adjustment on the reaction product prediction model, to enhance the robustness and generalization performance of the reaction product prediction model, and also has strong extendability for a larger data set, for performing a supervision task without relying on manual annotation.

Specifically, as indicated by the task Task4 shown in FIG. 15, reactants $R_{11} + R_{12} + R_{13}$ and a reaction product $P_{11}$ in one reaction array $R_{11} + R_{12} + R_{13} \rightarrow P_{11}$ are combined with reactants $R_{21} + R_{22} + R_{23}$ and a reaction product $P_{21}$ in another reaction array $R_{21} + R_{22} + R_{23} - P_{21}$, to obtain a composite reaction array, for example, $R_{11} + R_{12} + R_{13} + R_{21} + R_{22} + R_{23} \rightarrow P_{11} + P_{21}$.

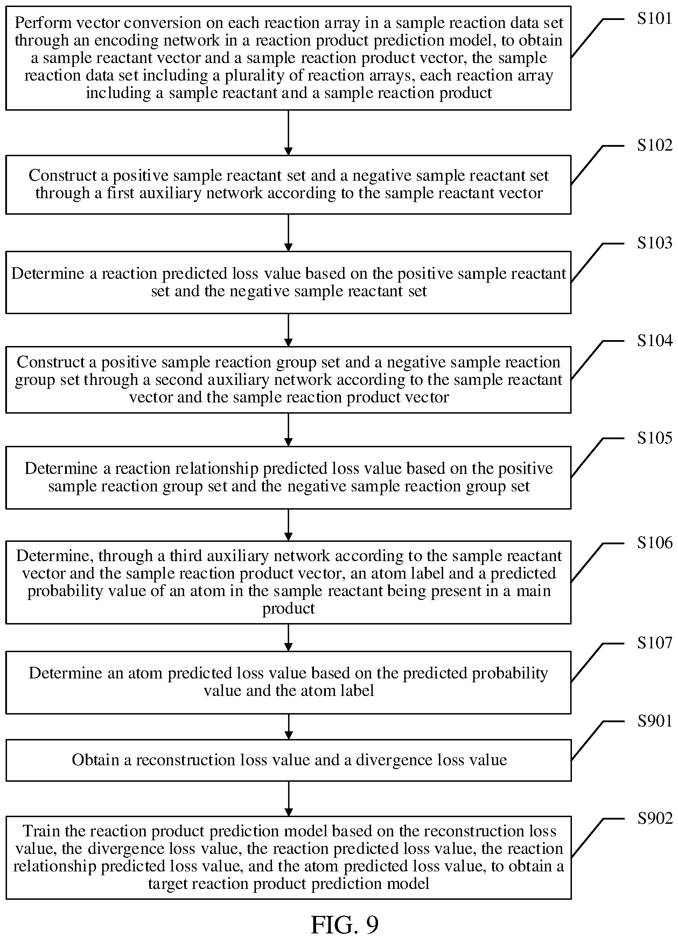

Optionally, based on the embodiment corresponding to FIG. 2, in another optional embodiment of the training method for a reaction product prediction model provided in this embodiment of this application, as shown in FIG. 9, the training the reaction product prediction model based on the reaction prediction error, the reaction relationship prediction error, and the atom prediction error, to obtain an updated reaction product prediction model, in step S108 includes the following steps:

Step S901: Obtain a reconstruction error and a divergence error.

Step S902: Train the reaction product prediction model by using the reconstruction error, the divergence error, the reaction prediction error, the reaction relationship prediction error, and the atom prediction error, to obtain the updated reaction product prediction model.

Specifically, when the VAE architecture satisfying electron transfer conservation shown in FIG. 16 is used as the reaction product prediction model, in this embodiment, a multi-task learning training method can be used, to obtain the reconstruction error and the divergence error based on the VAE architecture.

Further, objective functions, such as the reconstruction error, the divergence error, the reaction prediction error, the reaction relationship prediction error, and the atom prediction error, may be directly added to the total objective function in a weighted manner based on the following total objective function formula (6), to obtain a total objective function value:

$$L_{total} = L_{reconstruct} + \alpha L_{KL} + \beta L_A + \lambda L_B B + \theta L_C \quad (6)$$

where $L_{total}$ represents the total objective function value, $L_{reconstruct}$ represents the reconstruction error, $L_{KL}$ represents the divergence error, $L_A$ represents the reaction prediction error, $L_B$ represents the reaction relationship prediction error, $L_C$ represents the atom prediction error, and $\alpha$, $\beta$, $\lambda$, and $\theta$ are weight parameters set according to actual application requirements, which are not specifically limited herein.

Further, parameter adjustment may be performed on the reaction product prediction model based on the total objective function value. Specifically, parameter adjustment may be performed using an iterative method of backpropagation or another method, which is not specifically limited herein, to obtain the updated reaction product prediction model.

In this way, training the reaction product prediction model based on the reconstruction error, the divergence error, the reaction prediction error, the reaction relationship prediction error, and the atom prediction error helps the trained reaction product prediction model possess better performance, and also helps improve the efficiency of training the reaction product prediction model.

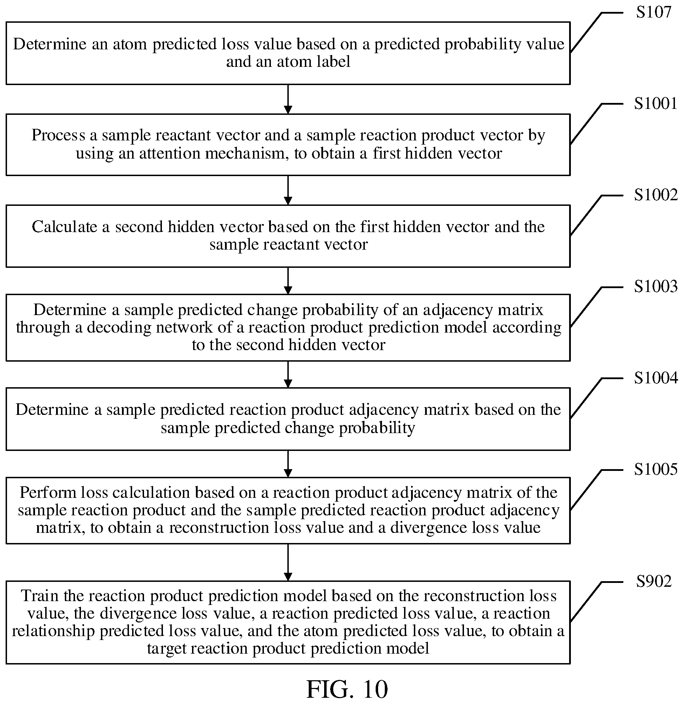

Optionally, based on the embodiment corresponding to FIG. 9, in another optional embodiment of the training method for a reaction product prediction model provided in this embodiment of this application, as shown in FIG. 10, the obtaining a reconstruction error and a divergence error in step S901 includes the following steps:

Step S1001: Process the sample reactant vector and the sample reaction product vector by using an attention mechanism, to obtain a first hidden vector.

Step S1002: Calculate a second hidden vector modes based on the first hidden vector and the sample reactant vector.

Step S1003: Determine a sample prediction change probability of an adjacency matrix using a decoding network of the reaction product prediction model according to the second hidden vector.

Step S1004: Determine a sample prediction reaction product adjacency matrix based on the sample predicted change probability.

Step S1005: Calculate based on a reaction product adjacency matrix of the sample reaction product and the sample prediction reaction product adjacency matrix, to obtain the reconstruction error and the divergence error.

Specifically, as shown in FIG. 16, an attention mechanism is adopted for the sample reactant vector and the sample reaction product vector. That is, a reactant embedding and a product embedding may be caused to pass through a layer of cross attention mechanism. Cross attention may be implemented directly through the transformer decoder based on the following formulas (7), (8), and (9), to obtain parameter vectors $\mu$ and log $\sigma$ of a Gaussian distribution, and in addition, a first hidden vector conforming to the Gaussian distribution is obtained through reparameterization:

$$h^z = Mean\big(TransformerDecoder(h^R, h^P)\big) \quad (7)$$

$$\mu = W_\mu ReLU(h^z) + b_\mu \quad (8)$$

$$\log \sigma = W_\sigma ReLU(h^z) + b_\sigma \quad (9)$$

where $h^z$ represents the first hidden vector, and $W_\mu$, $b_\mu$, $W_\sigma$ and $b_\sigma$ represent model parameters.

Further, a second hidden vector is calculated based on the first hidden vector and the sample reactant vector. Specifically, the first hidden vector and the sample reactant vector are added based on the following formula (10) and caused to pass through a layer of transformer, to obtain a new hidden vector, that is, the second hidden vector $h_L$:

$$h_L = Transformer(h^R + h^z). \quad (10)$$

Further, as shown in FIG. 16, the second hidden vector is inputted to the decoding network of the reaction product prediction model, and the second hidden vector $h_L$ is decoded through the decoding network. The decoding network decoder is mainly formed by two separate self-attention mechanisms. One self-attention mechanism outputs an attention matrix as a probability that electrons shared between two atoms increase, and the other self-attention mechanism outputs an attention matrix as a probability that electrons shared between two atoms decrease. Because transfer of electrons is conserved, this property is modeled using characteristics of the weight matrix of the attention mechanism, where specific decoding operations include the following formulas (11) and (12), to obtain a sample predicted change probability of the adjacency matrix:

$$W^{+d} = SelfAttention(h_L) \quad (11)$$

$$W^{-d} = SelfAttention(h_L) \quad (12)$$

where $W^{+d}$ represents a sample predicted change probability, that is, a probability matrix of electrons between each pair of atoms increasing, and $W^{-d}$ represents another sample predicted change probability, that is, a probability matrix of electrons between each pair of atoms decreasing.

Further, a sample predicted reaction product adjacency matrix is calculated based on the sample predicted change probability. Specifically, a sample predicted change amount of an adjacency matrix may be calculated based on the sample predicted change probability, the sample predicted reaction product adjacency matrix may be calculated based on the sample predicted change amount of the adjacency matrix and an adjacency matrix of a sample reactant, and then cross-entropy loss calculation may be performed based on the reaction product adjacency matrix of the sample reaction product and the sample predicted reaction product adjacency matrix, to obtain the reconstruction error and the divergence error.

Each value in an adjacency matrix in this embodiment is not just 0 or 1 (0 represents that there is no connection between corresponding atom pairs, and 1 represents that there is a connection between corresponding atom pairs), and instead, four values, 0, 1, 2, and 3 respectively represent no connection, a single-bond connection, a double-bond connection, and a triple-bond connection. An aromatic bond connection is represented as 1, and atoms connected by the aromatic bond connection are marked as aromatic atoms, for distinguishing from a single-bond connection.

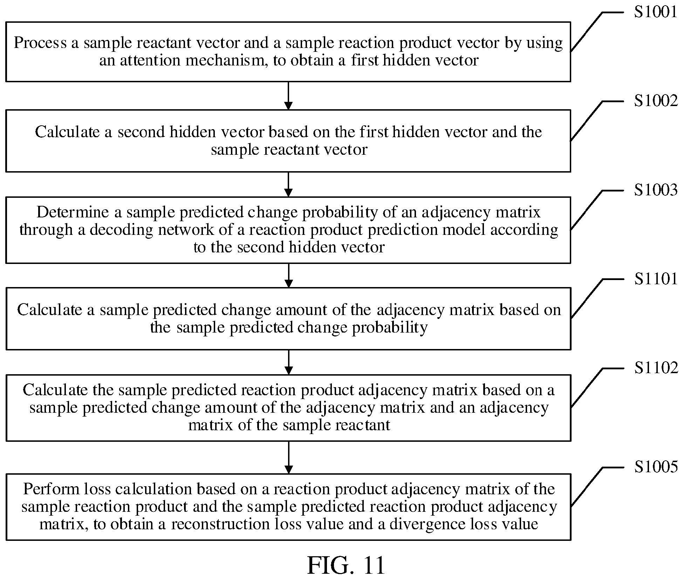

Optionally, based on the embodiment corresponding to FIG. 10, in another optional embodiment of the training method for a reaction product prediction model provided in this embodiment of this application, as shown in FIG. 11, the determining a sample predicted reaction product adjacency matrix based on the sample predicted change probability in step S1004 includes the following steps:

Step S1101: Calculate a sample prediction change amount of the adjacency matrix based on the sample prediction change probability.

Step S1102: Calculate the sample prediction reaction product adjacency matrix based on a sample prediction change amount of the adjacency matrix and an adjacency matrix of the sample reactant.

Specifically, after the sample predicted change probability is obtained, the sample predicted change amount of the adjacency matrix may be calculated based on the sample predicted change probability and using the following formula (13). That is, based on the probability matrix $W^{+d}$ of electrons between each pair of atoms increasing is subtracted from the probability matrix $W^{-d}$ of electrons between each pair of atoms decreasing and multiplied by 4, to obtain the predicted sample predicted change amount $\Delta A$ between each pair of atoms:

$$\Delta A = (W^{+d} - W^{-d}) \times 4 \quad (13)$$

where 4 represents a maximum change amount between each pair of atoms.

Further, the sample predicted reaction product adjacency matrix is calculated based on a sample predicted change amount of the adjacency matrix and an adjacency matrix of the sample reactant and using the following formula (14):

$$A^P = A^R + \Delta A \quad (14)$$

where $A^P$ represents the sample predicted reaction product adjacency matrix, and $A^R$ represents the adjacency matrix of the sample reactant.

Further, because the adjacency matrix needs to be symmetric, the sample predicted reaction product adjacency matrix may be symmetrized using the following formula (15):

$$A^P = \frac{A^P + (A^P)^T}{2}. \quad (15)$$

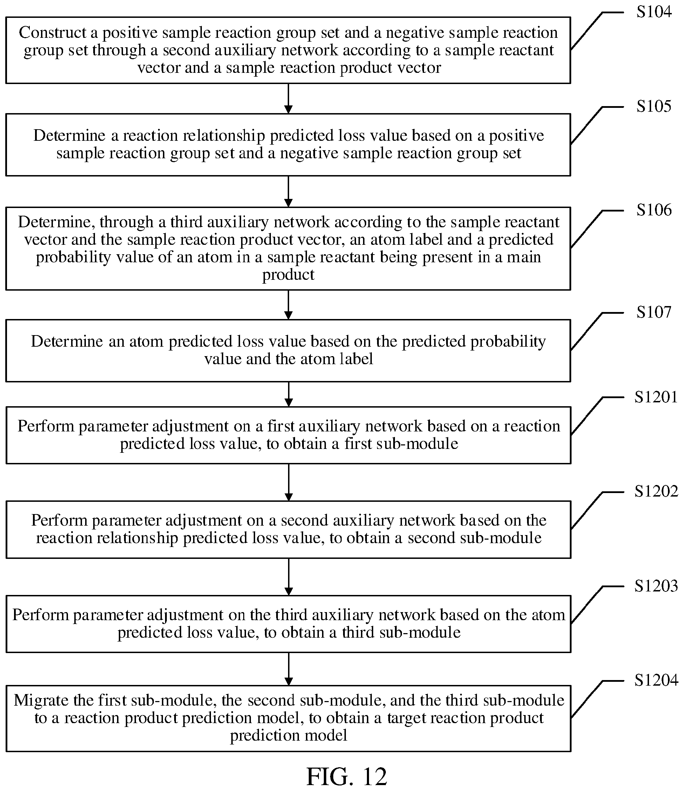

Optionally, based on the embodiment corresponding to FIG. 2, in another optional embodiment of the parameter adjustment method for a reaction product prediction model provided in this embodiment of this application, as shown in FIG. 12, the training the reaction product prediction model based on the reaction prediction error, the reaction relationship prediction error, and the atom prediction error, to obtain an updated reaction product prediction model, in step S108 includes the following steps:

Step S1201: Adjust parameters of the first auxiliary network based on the reaction prediction error, to obtain a first sub-module.

Step S1202: Adjusts parameters of the second auxiliary network based on the reaction relationship prediction error, to obtain a second sub-module.

Step S1203: Adjust parameters of the third auxiliary network based on the atom prediction error, to obtain a third sub-module.

Step S1204: Migrate the first sub-module, the second sub-module, and the third sub-module to the reaction product prediction model, to obtain the updated reaction product prediction model.

Specifically, in this embodiment, the first auxiliary network, the second auxiliary network, and the third auxiliary network may be pre-trained using a pre-training policy and based on the reaction prediction error, the reaction relationship prediction error, and the atom prediction error respectively, that is, parameter adjustment is performed on the first auxiliary network based on the reaction prediction error to obtain the first sub-module, parameter adjustment is performed on the second auxiliary network based on the reaction relationship prediction error to obtain the second sub-module, and parameter adjustment is performed on the third auxiliary network based on the atom prediction error to obtain the third sub-module, and then, the first sub-module, the second sub-module, and the third sub-module are migrated to the reaction product prediction model. Specifically, skeleton networks of the first sub-module, the second sub-module and the third sub-module is migrated to the reaction product prediction model, and then, layers outputting embeddings of the skeleton networks are connected to a decoder network layer for reaction product prediction, to obtain the updated reaction product prediction model.

In this way, the first auxiliary network, the second auxiliary network, and the third auxiliary network are pre-trained respectively based on the pre-training policy, and the trained first auxiliary network, the trained second auxiliary network, and the trained third auxiliary network are migrated to the reaction product prediction model, which can effectively improve the efficiency of training the reaction product prediction model, and also helps improve the performance of the reaction product prediction model.

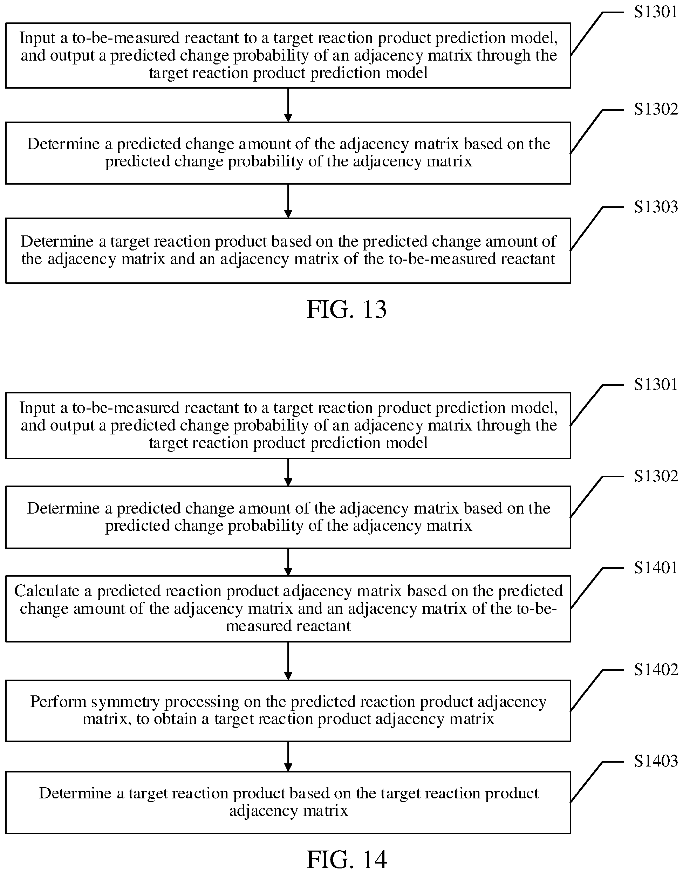

The application method for a reaction product prediction model according to the present disclosure is described below. The method may be performed by a computer device. The computer device may be a server or a terminal device. Referring to FIG. 13, an embodiment of the application method for a reaction product prediction model in this embodiment of the present disclosure includes the following steps:

Step S1301: Input an initial reactant to the updated reaction product prediction model, to output a predicted change probability of an adjacency matrix.

Step S1302: Predict a change amount of the adjacency matrix based on the predicted change probability of the adjacency matrix.

Step S1303: Determine a reaction product based on the predicted change amount of the adjacency matrix and an adjacency matrix of the initial reactant.

In this embodiment, after the to-be-measured reactant is obtained, the to-be-measured reactant may be inputted to the updated reaction product prediction model, the predicted change probability of the output adjacency matrix may be outputted through the updated reaction product prediction model, the predicted change amount of the adjacency matrix may be calculated based on the predicted change probability of the adjacency matrix, and then, the target reaction product may be determined based on the predicted change amount of the adjacency matrix and an adjacency matrix of the to-be-measured reactant, so that the obtained target reaction product can be subsequently applied to scenarios such as scientific pharmaceuticals or drug verification.

It may be understood that, the updated reaction product prediction model may be used as an effective verification tool for drug retrosynthesis, to improve the efficiency of researches on new drug synthesis paths. The updated reaction product prediction model may reveal some hidden scientific laws and provide new scientific knowledge. The updated reaction product prediction model may provide a prediction more accurate than those provided by experts, and not only can still predict a reliable candidate product when no known template can be used, but also can predict a new reaction, thereby greatly improving the efficiency of researching and development of new drugs and the like.

Specifically, the to-be-measured reactant is inputted to the updated reaction product prediction model, and the predicted change probability of the adjacency matrix is outputted through the updated reaction product prediction model, that is, based on the probability matrix $W^{+d}$ indicating electrons between each pair of atoms increase and the probability matrix $W^{-d}$ indicating electrons between each pair of atoms decrease, and then, the predicted change amount $\Delta A$ of the adjacency matrix may be calculated using the foregoing formula (13).

Optionally, based on the embodiment corresponding to FIG. 13, in another optional embodiment of the application method for a reaction product prediction model provided in this embodiment of this application, as shown in FIG. 14, the determining a target reaction product based on the predicted change amount of the adjacency matrix and an adjacency matrix of the to-be-measured reactant in step S1303 includes the following steps:

Step S1401: Calculate a prediction reaction product adjacency matrix based on the prediction change amount of the adjacency matrix and the adjacency matrix of the initial reactant.

Step S1402: Perform symmetry processing on the prediction reaction product adjacency matrix, to obtain a reaction product adjacency matrix.

Step S1403: Determine the reaction product based on the reaction product adjacency matrix.

Specifically, the predicted reaction product adjacency matrix may be calculated using the foregoing formula (14) and based on the predicted change amount of the adjacency matrix and an adjacency matrix of the to-be-measured reactant, where $A^P$ represents the predicted reaction product adjacency matrix, and $A^R$ represents the adjacency matrix of the to-be-measured reactant.

Further, because the adjacency matrix needs to symmetric, symmetry processing may be performed on the predicted reaction product adjacency matrix using the foregoing formula (15), to obtain the target reaction product adjacency matrix, and then, the target reaction product may be calculated based on the target reaction product adjacency matrix $A^P$.

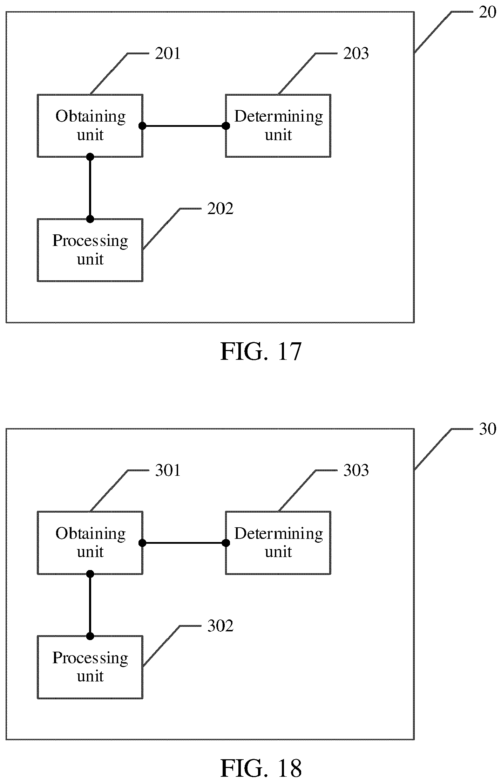

A training apparatus for a reaction product prediction model in the present disclosure is described below in detail. FIG. 17 is a schematic diagram of an embodiment of a training apparatus for a reaction product prediction model according to an embodiment of this application. A training apparatus 20 for a reaction product prediction model includes:

an obtaining unit 201, configured to perform vector conversion on a reaction array of a sample reaction data set using an encoding network of a reaction product prediction model, to obtain a sample reactant vector and a sample reaction product vector, the sample reaction data set indicating a plurality of reaction arrays each including a sample reactant and a sample reaction product,

the obtaining unit 201 being further configured to construct a positive sample reactant set and a negative sample reactant set using a first auxiliary network according to the sample reactant vector;

a processing unit 202, configured to determine a reaction prediction error based on the positive sample reactant set and the negative sample reactant set,

the obtaining unit 201 being further configured to construct a positive sample reaction pair set and a negative sample reaction pair set using a second auxiliary network according to the sample reactant vector and the sample reaction product vector;

the processing unit 202 being further configured to determine a reaction relationship prediction error based on the positive sample reaction pair set and the negative sample reaction pair set;

the obtaining unit 201 being further configured to predict, using a third auxiliary network and according to the sample reactant vector and the sample reaction product vector, an atom label and a probability value of an atom in the sample reactant being present in a main product; and

the processing unit 202 being further configured to determine an atom prediction error based on the predicted probability value and the predicted atom label; and

a determining unit 203, configured to train the reaction product prediction model by using the reaction prediction error, the reaction relationship prediction error, and the atom prediction error, to obtain an updated reaction product prediction model.

Optionally, based on the foregoing embodiment corresponding to FIG. 17, in another embodiment of the training apparatus for a reaction product prediction model provided in this embodiment of this application:

The processing unit 202 is further configured to perform data augmentation processing on the sample reaction data set, to obtain a sample composite reaction data set.

The processing unit 202 is further configured to combine the sample reaction data set and the sample composite reaction data set to form an extended sample reaction data set.

The obtaining unit 201 may be further configured to perform vector conversion on a reaction array in the extended sample reaction data set using the encoding network of the reaction product prediction model, to obtain the sample reactant vector and the sample reaction product vector.

Optionally, based on the foregoing embodiment corresponding to FIG. 17, in another embodiment of the training apparatus for a reaction product prediction model provided in this embodiment of this application, the obtaining unit 201 may be further configured to:

sample the sample reactant vectors corresponding to any two sample reactants from a same reaction as a positive sample reactants, and add the positive sample reactants to the positive sample reactant set; and

sample the sample reactant vectors corresponding to any two sample reactants from different reaction arrays as negative sample reactants, and add the negative sample reactants to the negative sample reactant set.

Optionally, based on the foregoing embodiment corresponding to FIG. 17, in another embodiment of the training apparatus for a reaction product prediction model provided in this embodiment of this application, the obtaining unit 201 may be further configured to:

use the sample reactant vectors and the sample reaction product vectors corresponding to all the reaction arrays as the positive sample reaction pair set; and

match the sample reactant vectors and the sample reaction product vectors corresponding to different reaction arrays, to obtain the negative sample reaction pair set.

Optionally, based on the foregoing embodiment corresponding to FIG. 17, in another embodiment of the training apparatus for a reaction product prediction model provided in this embodiment of this application, the obtaining unit 201 may be further configured to:

predict the sample reactant using the third auxiliary network, to obtain the probability value of the atom in the sample reactant being present in the main product;

compare the atom in the sample reactant with an atom in the sample reaction product based on the sample reactant vector and the sample reaction product vector, to obtain an atom comparison result; and

determine the atom label based on the atom comparison result.

Optionally, based on the foregoing embodiment corresponding to FIG. 17, in another embodiment of the training apparatus for a reaction product prediction model provided in this embodiment of this application, the obtaining unit 201 may be further configured to:

perform, for each reaction, interaction on internal information of each molecule of the sample reactant between atoms, to obtain sample reactant interaction information;

perform interaction between different sample reactants based on the sample reactant interaction information, to obtain the sample reactant vector;

perform, for each reaction, interaction on internal information of each molecule of the sample reaction product between atoms, to obtain sample reaction product interaction information; and

perform interaction between different sample reaction products based on the sample reaction product interaction information, to obtain the sample reaction product vector.

Optionally, based on the foregoing embodiment corresponding to FIG. 17, in another embodiment of the training apparatus for a reaction product prediction model provided in this embodiment of this application, the processing unit 202 may be further configured to:

select two reactions from the sample reaction data set;

combine sample reactants in the two selected reactions, to obtain a sample composite reactant;

combine sample reaction products in the two selected reactions, to obtain a sample composite reaction product; and

generate a composite reaction based on the sample composite reactant and the sample composite reaction product, and construct the sample composite reaction data set based on the composite reaction.

Optionally, based on the foregoing embodiment corresponding to FIG. 17, in another embodiment of the training apparatus for a reaction product prediction model provided in this embodiment of this application, the determining unit 203 may be further configured to:

obtain a reconstruction error and a divergence error; and

train the reaction product prediction model by using the reconstruction error, the divergence error, the reaction prediction error, the reaction relationship prediction error, and the atom prediction error, to obtain the updated reaction product prediction model.

Optionally, based on the foregoing embodiment corresponding to FIG. 17, in another embodiment of the training apparatus for a reaction product prediction model provided in this embodiment of this application, the determining unit 203 may be further configured to:

process the sample reactant vector and the sample reaction product vector by using an attention mechanism, to obtain a first hidden vector;

calculate a second hidden vector based on the first hidden vector and the sample reactant vector;

determine a sample prediction change probability of an adjacency matrix using a decoding network of the reaction product prediction model according to the second hidden vector;

determine a sample prediction reaction product adjacency matrix based on the sample prediction change probability; and

calculate based on a reaction product adjacency matrix of the sample reaction product and the sample predicted reaction product adjacency matrix, to obtain the reconstruction error and the divergence error.

Optionally, based on the foregoing embodiment corresponding to FIG. 17, in another embodiment of the training apparatus for a reaction product prediction model provided in this embodiment of this application, the determining unit 203 may be further configured to:

calculate a sample prediction change amount of the adjacency matrix based on the sample prediction change probability; and

calculate the sample prediction reaction product adjacency matrix based on a sample prediction change amount of the adjacency matrix and an adjacency matrix of the sample reactant.

Optionally, based on the foregoing embodiment corresponding to FIG. 17, in another embodiment of the training apparatus for a reaction product prediction model provided in this embodiment of this application, the determining unit 203 may be further configured to:

adjust parameters of the first auxiliary network based on the reaction prediction error, to obtain a first sub-module;

adjust parameters of the second auxiliary network based on the reaction relationship prediction error, to obtain a second sub-module;

adjust parameters of the third auxiliary network based on the atom prediction error, to obtain a third sub-module; and

migrate the first sub-module, the second sub-module, and the third sub-module to the reaction product prediction model, to obtain the updated reaction product prediction model.

An application apparatus for a reaction product prediction model in the present disclosure is described below in detail. FIG. 18 is a schematic diagram of an embodiment of an application apparatus for a reaction product prediction model according to an embodiment of this application. An application apparatus 30 for a reaction product prediction model includes:

an obtaining unit 301, configured to input an initial reactant to the updated reaction product prediction model, to output a predicted change probability of an adjacency matrix through the updated reaction product prediction model, the updated reaction product prediction model being obtained through training by the training apparatus for a reaction product prediction model shown in FIG. 17;

a processing unit 302, configured to predict a change amount of the adjacency matrix based on the predicted change probability of the adjacency matrix; and

a determining unit 303, configured to determine a target reaction product based on the predicted change amount of the adjacency matrix and an adjacency matrix of the initial reactant.

Optionally, based on the foregoing embodiment corresponding to FIG. 18, in another embodiment of the application apparatus for a reaction product prediction model provided in this embodiment of this application, the determining unit 303 may be further configured to:

calculate a prediction reaction product adjacency matrix based on the prediction change amount of the adjacency matrix and the adjacency matrix of the initial reactant;

perform symmetry processing on the prediction reaction product adjacency matrix, to obtain a reaction product adjacency matrix; and

determine the reaction product based on the reaction product adjacency matrix.

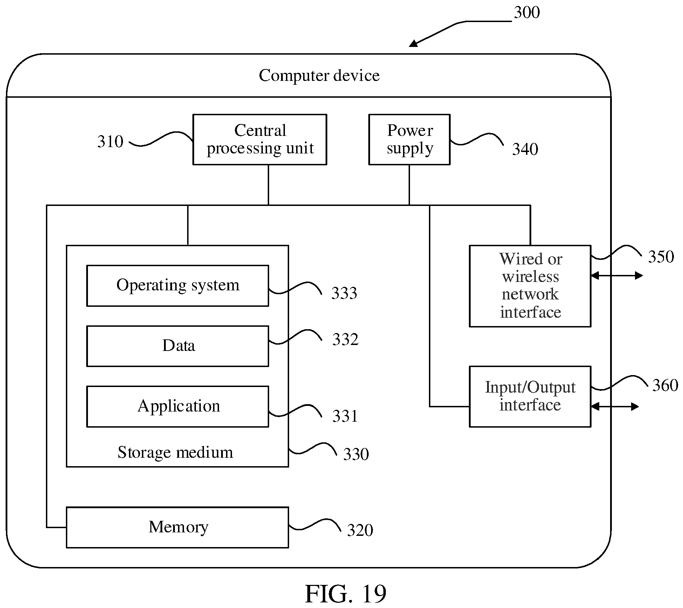

Another aspect of the present disclosure provides another schematic diagram of a computer device. FIG. 19 is a schematic structural diagram of a computer device according to an embodiment of this application. The computer device 300 may vary greatly due to different configurations or performance, and may include one or more central processing units (CPU) 310 (for example, one or more processors) and a memory 320, and one or more storage media 330 (for example, one or more mass storage devices) that store applications 331 or data 332. The memory 320 and the storage medium 330 may be transient or persistent storages. The program stored in the storage medium 330 may include one or more modules (not shown in the figure), and each module may include a series of instructions and operations for the computer device 300. Furthermore, the CPUs 310 may be configured to: communicate with the storage media 330, and perform, on the computer device 300, the series of instruction and operations in the storage medium 330.

The computer device 300 may further include one or more power supplies 340, one or more wired or wireless network interfaces 350, one or more input/output interfaces 360, and/or one or more operating systems 333, for example, Windows Server™, Mac OS X™, Unix™, Linux™, or FreeBSD™.

The foregoing computer device 300 is further configured to perform the steps in the embodiments corresponding to FIG. 2 to FIG. 13 and the steps in the embodiment corresponding to FIG. 14.

Another aspect of the present disclosure provides a computer-readable storage medium, having a computer program stored therein. When the computer program is executed by a processor, the steps in the method described in the embodiments shown in FIG. 2 to FIG. 13 and the steps in the method described in the embodiment shown in FIG. 14 are implemented.

Another aspect of the present disclosure provides a computer program product including a computer program. When the computer program is executed by a processor, the steps in the method described in the embodiments shown in FIG. 2 to FIG. 13 and the steps in the method described in the embodiment shown in FIG. 14 are implemented.

A person skilled in the art may clearly understand that, for the purpose of convenient and brief description, for a detailed working process of the system, apparatus, and unit described above, refer to a corresponding process in the method embodiments, and details are not described herein again.

In the several embodiments provided in this application, it is to be understood that the disclosed system, apparatus, and method may be implemented in other manners. For example, the described apparatus embodiment is merely an example. For example, the unit division is merely logical function division and may be other division during actual implementation. For example, a plurality of units or components may be combined or integrated into another system, or some features may be ignored or not performed. In addition, the displayed or discussed mutual couplings or direct couplings or communication connections may be implemented by using some interfaces. The indirect couplings or communication connections between the apparatuses or units may be implemented in electric, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments.

In addition, functional units in embodiments of the present disclosure may be integrated into one processing unit, or each of the units may exist alone physically, or two or more units are integrated into one unit. The integrated unit may be implemented in a form of hardware, or may be implemented in a form of a software functional unit.

When the integrated unit is implemented in the form of a software functional unit and sold or used as an independent product, the integrated unit may be stored in a computer-readable storage medium. Based on such an understanding, the technical solutions of the present disclosure essentially, or the part contributing to the conventional technology, or all or some of the technical solutions may be implemented in a form of a software product. The computer software product is stored in a storage medium, and includes several instructions for enabling a computer device (which may be a personal computer, a server, or a network device) to perform all or some of the steps of the methods described in embodiments of this application. The foregoing storage medium includes any medium that can store program code, for example, a USB flash drive, a removable hard disk, a read-only memory (ROM), a random access memory (RAM), a magnetic disk, or an optical disc.

**Claims**

1. A method for training a reaction product prediction model, executable by a computer device, the method comprising:

performing (S101) vector conversion on a reaction of a sample reaction data set using an encoding network of a reaction product prediction model, to obtain a sample reactant vector and a sample reaction product vector, the sample reaction data set indicating a plurality of reactions each comprising a sample reactant and a sample reaction product;

constructing (S102) a positive sample reactant set and a negative sample reactant set using a first auxiliary network according to the sample reactant vector;

determining (S103) a reaction prediction error based on the positive sample reactant set and the negative sample reactant set, the reaction prediction error indicating accuracy for predicting whether reactants inputted to the reaction product prediction model react;

constructing (S104) a positive sample reaction pair set and a negative sample reaction pair set using a second auxiliary network according to the sample reactant vector and the sample reaction product vector;

determining (S105) a reaction relationship prediction error based on the positive sample reaction pair set and the negative sample reaction pair set, the reaction relationship prediction error indicating accuracy for predicting whether a correspondence between a reactant and a reaction product is correct by the reaction product prediction model;

predicting (S106), using a third auxiliary network and according to the sample reactant vector and the sample reaction product vector, an atom label and a probability value of an atom in the sample reactant being present in a main product;

determining (S107) an atom prediction error based on the predicted probability value and the predicted atom label; and

training (S108) the reaction product prediction model by using the reaction prediction error, the reaction relationship prediction error, and the atom prediction error, to obtain an updated reaction product prediction model.

2. The method according to claim 1, wherein before the performing vector conversion on a reaction of a sample reaction data set using an encoding network of a reaction product prediction model, to obtain a sample reactant vector and a sample reaction product vector, the method further comprises:

performing data augmentation processing on the sample reaction data set, to obtain a sample composite reaction data set; and

combining the sample reaction data set and the sample composite reaction data set to form an extended sample reaction data set;

wherein the performing vector conversion on a reaction of a sample reaction data set using an encoding network of a reaction product prediction model, to obtain a sample reactant vector and a sample reaction product vector comprises:

performing vector conversion on a reaction in the extended sample reaction data set using the encoding network of the reaction product prediction model, to obtain the sample reactant vector and the sample reaction product vector.

3. The method according to claim 1 or 2, wherein the constructing a positive sample reactant set and a negative sample reactant set using a first auxiliary network according to the sample reactant vector comprises:

sampling the sample reactant vectors corresponding to any two sample reactants from a same reaction array as positive sample reactants, and adding the positive sample reactants to the positive sample reactant set; and sampling the sample reactant vectors corresponding to any two sample reactants from different reaction arrays as negative sample reactants, and adding the negative sample reactants to the negative sample reactant set.

4. The method according to any one of claims 1 to 3, wherein the constructing a positive sample reaction pair set and a negative sample reaction pair set using a second auxiliary network according to the sample reactant vector and the sample reaction product vector comprises:

using the sample reactant vectors and the sample reaction product vectors corresponding to all the reaction arrays as the positive sample reaction pair set; and

matching the sample reactant vectors and the sample reaction product vectors corresponding to different reaction arrays, to obtain the negative sample reaction pair set.

5. The method according to any one of claims 1 to 4, wherein the determining, using a third auxiliary network according to the sample reactant vector and the sample reaction product vector, an atom label and a predicted probability value of an atom in the sample reactant being present in a main product comprises:

predicting the sample reactant using the third auxiliary network, to obtain the probability value of the atom in the sample reactant being present in the main product;

comparing the atom in the sample reactant with an atom in the sample reaction product based on the sample reactant vector and the sample reaction product vector, to obtain an atom comparison result; and

determining the atom label based on the atom comparison result.

6. The method according to any one of claims 1 to 5, wherein the performing vector conversion on a reaction of a sample reaction data set using an encoding network of a reaction product prediction model, to obtain a sample reactant vector and a sample reaction product vector comprises:

performing, for each reaction, interaction on internal information of each molecule of the sample reactant between atoms, to obtain sample reactant interaction information;

performing interaction between different sample reactants based on the sample reactant interaction information, to obtain the sample reactant vector;

performing, for each reaction, interaction on internal information of each molecule of the sample reaction product between atoms, to obtain sample reaction product interaction information; and

performing interaction between different sample reaction products based on the sample reaction product interaction information, to obtain the sample reaction product vector.

7. The method according to claim 2, wherein the performing data augmentation processing on the sample reaction data set, to obtain a sample composite reaction data set comprises:

selecting two reactions from the sample reaction data set;

combining sample reactants in the two selected reactions, to obtain a sample composite reactant;

combining sample reaction products in the two selected reactions, to obtain a sample composite reaction product; and

generating a composite reaction based on the sample composite reactant and the sample composite reaction product, and constructing the sample composite reaction data set based on the composite reaction.

8. The method according to any one of claims 1 to 6, wherein the training the reaction product prediction model by using the reaction prediction error, the reaction relationship prediction error, and the atom prediction error, to obtain an updated reaction product prediction model comprises:

obtaining a reconstruction error and a divergence error; and

training the reaction product prediction model by using the reconstruction error, the divergence error, the reaction prediction error, the reaction relationship prediction error, and the atom prediction error, to obtain the updated reaction product prediction model.

9. The method according to claim 8, wherein the obtaining a reconstruction error and a divergence error comprises:

processing the sample reactant vector and the sample reaction product vector by using an attention mechanism, to obtain a first hidden vector;

calculating a second hidden vector based on the first hidden vector and the sample reactant vector;

determining a sample prediction change probability of an adjacency matrix using a decoding network of the reaction product prediction model according to the second hidden vector;

determining a sample prediction reaction product adjacency matrix based on the sample prediction change probability; and

calculating, based on a reaction product adjacency matrix of the sample reaction product and the sample prediction reaction product adjacency matrix, to obtain the reconstruction error and the divergence error.

10. The method according to claim 9, wherein the determining a sample prediction reaction product adjacency matrix based on the sample prediction change probability comprises:

calculating a sample prediction change amount of the adjacency matrix based on the sample prediction change probability; and

calculating the sample prediction reaction product adjacency matrix based on a sample prediction change amount of the adjacency matrix and an adjacency matrix of the sample reactant.

11. The method according to any one of claims 1 to 10, wherein the training the reaction product prediction model by using the reaction prediction error, the reaction relationship prediction error, and the atom prediction error, to obtain an updated reaction product prediction model comprises:

adjusting parameters of the first auxiliary network based on the reaction prediction error, to obtain a first sub-module;
adjusting parameters of the second auxiliary network based on the reaction relationship prediction error, to obtain a second sub-module;
adjusting parameters of the third auxiliary network based on the atom prediction error, to obtain a third sub-module; and
migrating the first sub-module, the second sub-module, and the third sub-module to the reaction product prediction model, to obtain the updated reaction product prediction model.

12. A method for applying a reaction product prediction model, executable by a computer device, the method comprising:

inputting (S1301) an initial reactant to the updated reaction product prediction model according to any one of claims 1 to 11, to output a predicted change probability of an adjacency matrix;
predicting (S1302) a change amount of the adjacency matrix based on the predicted change probability of the adjacency matrix; and
determining (S1303) a reaction product based on the predicted change amount of the adjacency matrix and an adjacency matrix of the initial reactant.

13. The method according to claim 12, wherein the determining a reaction product based on the prediction change amount of the adjacency matrix and an adjacency matrix of the initial reactant comprises:

calculating a prediction reaction product adjacency matrix based on the prediction change amount of the adjacency matrix and the adjacency matrix of the initial reactant;
performing symmetry processing on the prediction reaction product adjacency matrix, to obtain a reaction product adjacency matrix; and
determining the reaction product based on the reaction product adjacency matrix.

14. A training apparatus for a reaction product prediction model, comprising:

an obtaining unit, configured to perform vector conversion on each reaction array in a sample reaction data set through an encoding network in a reaction product prediction model, to obtain a sample reactant vector and a sample reaction product vector, the sample reaction data set comprising a plurality of reaction arrays, each reaction array comprising a sample reactant and a sample reaction product,
the obtaining unit being further configured to construct a positive sample reactant set and a negative sample reactant set through a first auxiliary network according to the sample reactant vector;
a processing unit, configured to determine a reaction predicted loss value based on the positive sample reactant set and the negative sample reactant set,
the obtaining unit being configured to construct a positive sample reaction group set and a negative sample reaction group set through a second auxiliary network according to the sample reactant vector and the sample reaction product vector;
the processing unit being further configured to determine a reaction relationship predicted loss value based on the positive sample reaction group set and the negative sample reaction group set;
the obtaining unit being configured to determine, through a third auxiliary network according to the sample reactant vector and the sample reaction product vector, an atom label and a predicted probability value of an atom in the sample reactant being present in a main product; and
the processing unit being further configured to determine an atom predicted loss value based on the predicted probability value and the atom label; and
a determining unit, configured to train the reaction product prediction model based on the reaction predicted loss value, the reaction relationship predicted loss value, and the atom predicted loss value, to obtain a target reaction product prediction model.

15. An application apparatus for a reaction product prediction model, comprising:

an obtaining unit, configured to input a to-be-measured reactant to a target reaction product prediction model, and output a predicted change probability of an adjacency matrix through the target reaction product prediction model;

a processing unit, configured to determine a predicted change amount of the adjacency matrix based on the predicted change probability of the adjacency matrix; and

a determining unit, configured to determine a target reaction product based on the predicted change amount of the adjacency matrix and an adjacency matrix of the to-be-measured reactant.

16. A computer device, comprising a memory, a processor, and a bus system, wherein the memory stores a computer program, the processor is configured to execute the computer program to implement the method according to any one of claims 1 to 11 and the method according to claim 12 or 13; and

the bus system is configured to connect the memory and the processor, to cause the memory and the processor to perform communication.

17. A computer-readable storage medium storing a computer program, wherein the computer program is executed by a processor to implement the method according to any one of claims 1 to 11 and the method according to claim 12 or 13.

18. A computer program product, comprising a computer program, the computer program, when executed by a processor, implementing the operations of the method according to any one of claims 1 to 11 and the operations of the method according to claim 12 or 13.

Server

Network

Terminal device

FIG. 1

Perform vector conversion on each reaction array in a sample reaction data set through an encoding network in a reaction product prediction model, to obtain a sample reactant vector and a sample reaction product vector, the sample reaction data set including a plurality of reaction arrays, each reaction array including a sample reactant and a sample reaction product

S101

Construct a positive sample reactant set and a negative sample reactant set through a first auxiliary network according to the sample reactant vector

S102

Determine a reaction predicted loss value based on the positive sample reactant set and the negative sample reactant set

S103

Construct a positive sample reaction group set and a negative sample reaction group set through a second auxiliary network according to the sample reactant vector and the sample reaction product vector

S104

Determine a reaction relationship predicted loss value based on the positive sample reaction group set and the negative sample reaction group set

S105

Determine, through a third auxiliary network according to the sample reactant vector and the sample reaction product vector, an atom label and a predicted probability value of an atom in the sample reactant being present in a main product

S106

Determine an atom predicted loss value based on the predicted probability value and the atom label

S107

Train the reaction product prediction model based on the reaction predicted loss value, the reaction relationship predicted loss value, and the atom predicted loss value, to obtain a target reaction product prediction model

S108

FIG. 2

S301

Perform data augmentation processing on a sample reaction data set, to obtain a sample composite reaction data set

S302

Summarize the sample reaction data set and the sample composite reaction data set into an extended sample reaction data set

S303

Perform vector conversion on each reaction array in the extended sample reaction data set through an encoding network in a reaction product prediction model, to obtain a sample reactant vector and a sample reaction product vector

S102

Construct a positive sample reactant set and a negative sample reactant set through a first auxiliary network according to the sample reactant vector

S103

Determine a reaction predicted loss value based on the positive sample reactant set and the negative sample reactant set

S104

Construct a positive sample reaction group set and a negative sample reaction group set through a second auxiliary network according to the sample reactant vector and the sample reaction product vector

S105

Determine a reaction relationship predicted loss value based on the positive sample reaction group set and the negative sample reaction group set

S106

Determine, through a third auxiliary network according to the sample reactant vector and the sample reaction product vector, an atom label and a predicted probability value of an atom in the sample reactant being present in a main product

S107

Determine an atom predicted loss value based on the predicted probability value and the atom label

S108

Train the reaction product prediction model based on the reaction predicted loss value, the reaction relationship predicted loss value, and the atom predicted loss value, to obtain a target reaction product prediction model

FIG. 3

Sample sample reactant vectors corresponding to any two sample reactants from a same reaction array as a positive sample reactant combination, and add the positive sample reactant combination to a positive sample reactant set ⟋⎯ S401

Sample sample reactant vectors corresponding to any two sample reactants from different reaction arrays as a negative sample reactant combination, and add the negative sample reactant combination to a negative sample reactant set ⟋⎯ S402

Determine a reaction predicted loss value based on the positive sample reactant set and the negative sample reactant set ⟋⎯ S103

Construct a positive sample reaction group set and a negative sample reaction group set through a second auxiliary network according to a sample reactant vector and a sample reaction product vector ⟋⎯ S104

Determine a reaction relationship predicted loss value based on the positive sample reaction group set and the negative sample reaction group set ⟋⎯ S105

Determine, through a third auxiliary network according to the sample reactant vector and the sample reaction product vector, an atom label and a predicted probability value of an atom in a sample reactant being present in a main product ⟋⎯ S106

Determine an atom predicted loss value based on the predicted probability value and the atom label ⟋⎯ S107

Train a reaction product prediction model based on the reaction predicted loss value, the reaction relationship predicted loss value, and the atom predicted loss value, to obtain a target reaction product prediction model ⟋⎯ S108

FIG. 4

```
┌────────────────────────────────────────────────────────────┐       ┌─ S103
│ Determine a reaction predicted loss value based on a         │      │
│ positive sample reactant set and a negative sample reactant  │
│ set                                                          │
└────────────────────────────────────────────────────────────┘

┌────────────────────────────────────────────────────────────┐       ┌─ S501
│ Use sample reactant vectors and sample reaction product      │      │
│ vectors corresponding to all reaction arrays as the positive │
│ sample reaction group set                                    │
└────────────────────────────────────────────────────────────┘

┌────────────────────────────────────────────────────────────┐       ┌─ S502
│ Match sample reactant vectors and sample reaction product    │      │
│ vectors corresponding to different reaction arrays, to       │
│ obtain the negative sample reaction group set                │
└────────────────────────────────────────────────────────────┘

┌────────────────────────────────────────────────────────────┐       ┌─ S104
│ Construct a positive sample reaction group set and a         │      │
│ negative sample reaction group set through a second          │
│ auxiliary network according to a sample reactant vector and  │
│ a sample reaction product vector                             │
└────────────────────────────────────────────────────────────┘

┌────────────────────────────────────────────────────────────┐       ┌─ S105
│ Determine a reaction relationship predicted loss value based │      │
│ on the positive sample reaction group set and the negative   │
│ sample reaction group set                                    │
└────────────────────────────────────────────────────────────┘

┌────────────────────────────────────────────────────────────┐       ┌─ S106
│ Determine, through a third auxiliary network according to    │      │
│ the sample reactant vector and the sample reaction product   │
│ vector, an atom label and a predicted probability value of   │
│ an atom in a sample reactant being present in a main product │
└────────────────────────────────────────────────────────────┘

┌────────────────────────────────────────────────────────────┐       ┌─ S107
│ Determine an atom predicted loss value based on the          │      │
│ predicted probability value and the atom label               │
└────────────────────────────────────────────────────────────┘

┌────────────────────────────────────────────────────────────┐       ┌─ S108
│ Train a reaction product prediction model based on the       │      │
│ reaction predicted loss value, the reaction relationship     │
│ predicted loss value, and the atom predicted loss value, to  │
│ obtain a target reaction product prediction model            │
└────────────────────────────────────────────────────────────┘
```

FIG. 5

Determine a reaction relationship predicted loss value based on a positive sample reaction group set and a negative sample reaction group set
└─ S105

Predict a sample reactant through a third auxiliary network, to obtain a predicted probability value of an atom in a sample reactant being present in a main product
└─ S601

Compare the atom in the sample reactant and an atom in a sample reaction product based on a sample reactant vector and a sample reaction product vector, to obtain an atom comparison result
└─ S602

Determine an atom label based on the atom comparison result
└─ S603

Determine an atom predicted loss value based on the predicted probability value and the atom label
└─ S107

Train a reaction product prediction model based on a reaction predicted loss value, the reaction relationship predicted loss value, and the atom predicted loss value, to obtain a target reaction product prediction model
└─ S108

FIG. 6

Perform, for each reaction array, interaction on internal information of each molecule of a sample reactant between atoms, to obtain sample reactant interaction information — S701

Perform interaction between different sample reactants based on the sample reactant interaction information, to obtain a sample reactant vector — S702

Perform, for each reaction array, interaction on internal information of each molecule of a sample reaction product between atoms, to obtain sample reaction product interaction information — S703

Perform interaction between different sample reaction products based on the sample reaction product interaction information, to obtain a sample reaction product vector — S704

Construct a positive sample reactant set and a negative sample reactant set through a first auxiliary network according to the sample reactant vector — S102

Determine a reaction predicted loss value based on the positive sample reactant set and the negative sample reactant set — S103

Construct a positive sample reaction group set and a negative sample reaction group set through a second auxiliary network according to the sample reactant vector and the sample reaction product vector — S104

FIG. 7

Select two reaction arrays randomly from a sample reaction data set — S801

Combine sample reactants in the two selected reaction arrays, to obtain a sample composite reactant — S802

Combine sample reaction products in the two selected reaction arrays, to obtain a sample composite reaction product — S803

Obtain a composite reaction array based on the sample composite reactant and the sample composite reaction product, and construct a sample composite reaction data set based on the composite reaction array — S804

Summarize the sample reaction data set and the sample composite reaction data set into an extended sample reaction data set — S302

Perform vector conversion on each reaction array in the extended sample reaction data set through an encoding network in a reaction product prediction model, to obtain a sample reactant vector and a sample reaction product vector — S303

FIG. 8

Perform vector conversion on each reaction array in a sample reaction data set through an encoding network in a reaction product prediction model, to obtain a sample reactant vector and a sample reaction product vector, the sample reaction data set including a plurality of reaction arrays, each reaction array including a sample reactant and a sample reaction product — S101

Construct a positive sample reactant set and a negative sample reactant set through a first auxiliary network according to the sample reactant vector — S102

Determine a reaction predicted loss value based on the positive sample reactant set and the negative sample reactant set — S103

Construct a positive sample reaction group set and a negative sample reaction group set through a second auxiliary network according to the sample reactant vector and the sample reaction product vector — S104

Determine a reaction relationship predicted loss value based on the positive sample reaction group set and the negative sample reaction group set — S105

Determine, through a third auxiliary network according to the sample reactant vector and the sample reaction product vector, an atom label and a predicted probability value of an atom in the sample reactant being present in a main product — S106

Determine an atom predicted loss value based on the predicted probability value and the atom label — S107

Obtain a reconstruction loss value and a divergence loss value — S901

Train the reaction product prediction model based on the reconstruction loss value, the divergence loss value, the reaction predicted loss value, the reaction relationship predicted loss value, and the atom predicted loss value, to obtain a target reaction product prediction model — S902

FIG. 9

Determine an atom predicted loss value based on a predicted probability value and an atom label — S107

Process a sample reactant vector and a sample reaction product vector by using an attention mechanism, to obtain a first hidden vector — S1001

Calculate a second hidden vector based on the first hidden vector and the sample reactant vector — S1002

Determine a sample predicted change probability of an adjacency matrix through a decoding network of a reaction product prediction model according to the second hidden vector — S1003

Determine a sample predicted reaction product adjacency matrix based on the sample predicted change probability — S1004

Perform loss calculation based on a reaction product adjacency matrix of the sample reaction product and the sample predicted reaction product adjacency matrix, to obtain a reconstruction loss value and a divergence loss value — S1005

Train the reaction product prediction model based on the reconstruction loss value, the divergence loss value, a reaction predicted loss value, a reaction relationship predicted loss value, and the atom predicted loss value, to obtain a target reaction product prediction model — S902

FIG. 10

Process a sample reactant vector and a sample reaction product vector by using an attention mechanism, to obtain a first hidden vector — S1001

Calculate a second hidden vector based on the first hidden vector and the sample reactant vector — S1002

Determine a sample predicted change probability of an adjacency matrix through a decoding network of a reaction product prediction model according to the second hidden vector — S1003

Calculate a sample predicted change amount of the adjacency matrix based on the sample predicted change probability — S1101

Calculate the sample predicted reaction product adjacency matrix based on a sample predicted change amount of the adjacency matrix and an adjacency matrix of the sample reactant — S1102

Perform loss calculation based on a reaction product adjacency matrix of the sample reaction product and the sample predicted reaction product adjacency matrix, to obtain a reconstruction loss value and a divergence loss value — S1005

FIG. 11

Construct a positive sample reaction group set and a negative sample reaction group set through a second auxiliary network according to a sample reactant vector and a sample reaction product vector ⟶ S104

Determine a reaction relationship predicted loss value based on a positive sample reaction group set and a negative sample reaction group set ⟶ S105

Determine, through a third auxiliary network according to the sample reactant vector and the sample reaction product vector, an atom label and a predicted probability value of an atom in a sample reactant being present in a main product ⟶ S106

Determine an atom predicted loss value based on the predicted probability value and the atom label ⟶ S107

Perform parameter adjustment on a first auxiliary network based on a reaction predicted loss value, to obtain a first sub-module ⟶ S1201

Perform parameter adjustment on a second auxiliary network based on the reaction relationship predicted loss value, to obtain a second sub-module ⟶ S1202

Perform parameter adjustment on the third auxiliary network based on the atom predicted loss value, to obtain a third sub-module ⟶ S1203

Migrate the first sub-module, the second sub-module, and the third sub-module to a reaction product prediction model, to obtain a target reaction product prediction model ⟶ S1204

FIG. 12

Input a to-be-measured reactant to a target reaction product prediction model, and output a predicted change probability of an adjacency matrix through the target reaction product prediction model — S1301

Determine a predicted change amount of the adjacency matrix based on the predicted change probability of the adjacency matrix — S1302

Determine a target reaction product based on the predicted change amount of the adjacency matrix and an adjacency matrix of the to-be-measured reactant — S1303

## FIG. 13

Input a to-be-measured reactant to a target reaction product prediction model, and output a predicted change probability of an adjacency matrix through the target reaction product prediction model — S1301

Determine a predicted change amount of the adjacency matrix based on the predicted change probability of the adjacency matrix — S1302

Calculate a predicted reaction product adjacency matrix based on the predicted change amount of the adjacency matrix and an adjacency matrix of the to-be-measured reactant — S1401

Perform symmetry processing on the predicted reaction product adjacency matrix, to obtain a target reaction product adjacency matrix — S1402

Determine a target reaction product based on the target reaction product adjacency matrix — S1403

## FIG. 14

FIG. 15

FIG. 16

20

201        203

Obtaining
unit

Determining
unit

202

Processing
unit

FIG. 17

30

301        303

Obtaining
unit

Determining
unit

302

Processing
unit

FIG. 18

FIG. 19

## INTERNATIONAL SEARCH REPORT

| International application No. |
|---|
| **PCT/CN2023/089401** |

| A. | CLASSIFICATION OF SUBJECT MATTER |
|---|---|

G06N3/04(2023.01)i

According to International Patent Classification (IPC) or to both national classification and IPC

| B. | FIELDS SEARCHED |
|---|---|

Minimum documentation searched (classification system followed by classification symbols)

IPC: G06N

Documentation searched other than minimum documentation to the extent that such documents are included in the fields searched

Electronic data base consulted during the international search (name of data base and, where practicable, search terms used)

VEN, CNABS, CNTXT, WOTXT, EPTXT, USTXT, CNKI, IEEE: 反应, 产物, 向量, 正样本, 负样本, 损失, 关系, 原子, 概率, 标签, 训练, process, outcome, vector, positive sample, negative sample, loss, relation, atom, probability, label, train

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 115204370 A (TENCENT TECHNOLOGY (SHENZHEN) CO., LTD.) 18 October 2022 (2022-10-18)<br>  claims 1-18 | 1-18 |
| A | CN 114464267 A (TENCENT TECHNOLOGY (SHENZHEN) CO., LTD.) 10 May 2022 (2022-05-10)<br>  description, paragraphs [0104]-[0236] | 1-18 |
| A | CN 113838536 A (YANTAI GOGETTER INTELLIGENT TECHNOLOGY CO., LTD.) 24 December 2021 (2021-12-24)<br>  entire document | 1-18 |
| A | EP 3945526 A1 (SAMSUNG ELECTRONICS CO., LTD.) 02 February 2022 (2022-02-02)<br>  entire document | 1-18 |
| A | WO 2022012407 A1 (HUAWEI TECHNOLOGIES CO., LTD.) 20 January 2022 (2022-01-20)<br>  entire document | 1-18 |

☐ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **17 May 2023** | **23 May 2023** |

| Name and mailing address of the ISA/CN | Authorized officer |
|---|---|
| **China National Intellectual Property Administration (ISA/ CN)**<br>**China No. 6, Xitucheng Road, Jimenqiao, Haidian District, Beijing 100088** | |
| | Telephone No. |

Form PCT/ISA/210 (second sheet) (July 2022)

INTERNATIONAL SEARCH REPORT
Information on patent family members

International application No.

**PCT/CN2023/089401**

| Patent document cited in search report | | | Publication date (day/month/year) | Patent family member(s) | | | Publication date (day/month/year) |
|---|---|---|---|---|---|---|---|
| CN | 115204370 | A | 18 October 2022 | None | | | |
| CN | 114464267 | A | 10 May 2022 | HK | 40070820 | A0 | 04 November 2022 |
| CN | 113838536 | A | 24 December 2021 | None | | | |
| EP | 3945526 | A1 | 02 February 2022 | JP | 2022027571 | A | 10 February 2022 |
| | | | | US | 2022036182 | A1 | 03 February 2022 |
| | | | | CN | 114093430 | A | 25 February 2022 |
| | | | | KR | 20220014798 | A | 07 February 2022 |
| WO | 2022012407 | A1 | 20 January 2022 | EP | 4167130 | A1 | 19 April 2023 |

Form PCT/ISA/210 (patent family annex) (July 2022)

**REFERENCES CITED IN THE DESCRIPTION**

**Patent documents cited in the description**

- CN 2022108264623